# EUROPEAN PATENT SPECIFICATION

(11) **EP 4 330 982 B1**
(45) Date of publication and mention of the grant of the patent: **17.06.2026**
(21) Application number: 23795625.5
(22) Date of filing: 28.04.2023
(51) Int. Cl.: G16H 30/20, G16H 30/40, G16H 50/20

(54) **METHODS AND SYSTEMS FOR IMAGING AND IMAGE DATA PROCESSING**
VERFAHREN UND SYSTEME ZUR BILDGEBUNG UND BILDDATENVERARBEITUNG
PROCÉDÉS ET SYSTÈMES D'IMAGERIE ET DE TRAITEMENT DE DONNÉES D'IMAGE

(30) Priority: 29.04.2022 CN 202210465380
(43) Date of publication of application: 06.03.2024
(73) Proprietor: Shanghai United Imaging Healthcare Co., Ltd., Shanghai 201807 (CN)
(72) Inventor: LIU, Zhantao, Shanghai 201807 (CN)
(74) Representative: Wang, Bo
(86) International application number: PCT/CN2023/091586
(87) International publication number: WO 2023/208193

(56) References cited:
- CN-A- 102 201 029
- CN-A- 111 640 111
- CN-A- 112 826 493
- CN-A- 114 864 053
- US-A1- 2008 009 723
- US-A1- 2017 224 260

## Description

### CROSS-REFERENCE TO RELATED APPLICATIONS

This application claims priority to Chinese Patent Application No. 202210465380.0, filed on April 29, 2022.

### TECHNICAL FIELD

The present disclosure relates to the field of imaging, in particular, to methods and systems for imaging and image data processing.

### BACKGROUND

The technology of medical imaging can be used as a medical auxiliary tool for diagnosis and treatment. Taking the technology of magnetic resonance imaging (MRI) as an example, with the development of the magnet technology, the imaging technology, the image processing technology and other technologies, the MRI has made a rapid development and has become one of the main methods of routine imaging examinations.

At present, the MRI is mainly used for the detection and diagnosis of heart diseases, vascular diseases, thoracic and abdominal organ diseases, etc. However, in the practical applications, the magnetic resonance scanning is often affected by various factors, resulting in uneven quality of scanned images. To address the problem of uneven scanned image quality, relevant prior art US 2008/009723 A1 discloses a method and system for synchronously obtaining physiological signal data in medical imaging, the method comprising: acquiring a patient's physiological signal data in real time (e.g., hemodynamic or electrophysiological data) via auxiliary devices (e.g., a blood pressure cuff, an SPO2 monitor, an electrocardiograph); synchronizing the physiological signal data with the scanning process of medical imaging equipment (e.g., ultrasound imaging equipment); aligning time stamps of the physiological signal data and imaging data to ensure their temporal correlation; and displaying physiological signal data in the form of waveform graphs, etc., to assist a physician in monitoring the patient's status in real time. It should be noted that US 2008/009723 A1 only reflects the patient's vital signs in real time and cannot use historical data and images to display the patient's physiological signals within a certain period in the past. Specifically, US 2008/009723 A1 is unable to analyze the patient's physiological signals within a specific historical event time period or a time period of a certain protocol scan.

Therefore, it is necessary to utilize historical physiological data and images associated with imaging data during the imaging process for image quality analysis, thereby improving image quality.

### SUMMARY

The invention is set out in the appended set of claims.

### BRIEF DESCRIPTION OF THE DRAWINGS

The present disclosure is further illustrated in terms of exemplary embodiments, and these exemplary embodiments are described in detail with reference to the drawings. These embodiments are not restrictive. In these embodiments, the same number indicates the same structure, wherein:
FIG. 1 is a schematic diagram illustrating an exemplary application scenario of an imaging system according to some embodiments of the present disclosure;
FIG. 2 is a block diagram illustrating an exemplary imaging system according to some embodiments of the present disclosure;
FIG. 3 is a flowchart illustrating an exemplary process of imaging according to some embodiments of the present disclosure;
FIG. 4 is a block diagram illustrating an exemplary system for image data processing according to some embodiments of the present disclosure;
FIG. 5 is a flowchart illustrating an exemplary process for image data processing according to some embodiments of the present disclosure;
FIG. 6A is a schematic diagram illustrating an exemplary process for storing physiological feature data according to some embodiments of the present disclosure;
FIG. 6B is a schematic diagram illustrating an exemplary process for calling physiological feature data according to some embodiments of the present disclosure;
FIGs. 7A and 7B are schematic diagrams illustrating exemplary application scenarios of a method for data processing according to some embodiments of the present disclosure;
FIG. 8 is a flowchart illustrating an exemplary process for data processing according to some embodiments of the present disclosure;
FIG. 9 is a schematic diagram illustrating exemplary physiological feature image data representing respiratory data of an object according to some embodiments of the present disclosure;
FIG. 10 is another schematic diagram illustrating exemplary physiological feature image data representing respiratory data of an object according to some embodiments of the present disclosure;
FIG. 11 is another flowchart illustrating an exemplary process for data processing according to some embodiments of the present disclosure;
FIG. 12 is another flowchart illustrating an exemplary process for data processing according to some embodiments of the present disclosure;
FIG. 13 is another flowchart illustrating an exemplary process for data processing according to some embodiments of the present disclosure;
FIG. 14 is a schematic diagram illustrating an exemplary interface of a rendering selection controller according to some embodiments of the present disclosure;
FIG. 15 is a block diagram illustrating a structure of an exemplary data processing device according to some embodiments of the present disclosure;
FIG. 16 is a schematic diagram illustrating internal structures of an exemplary computing device according to some embodiments of the present disclosure; and
FIG. 17 is another schematic diagram illustrating internal structures of an exemplary computing device according to some embodiments of the present disclosure.

### DETAILED DESCRIPTION

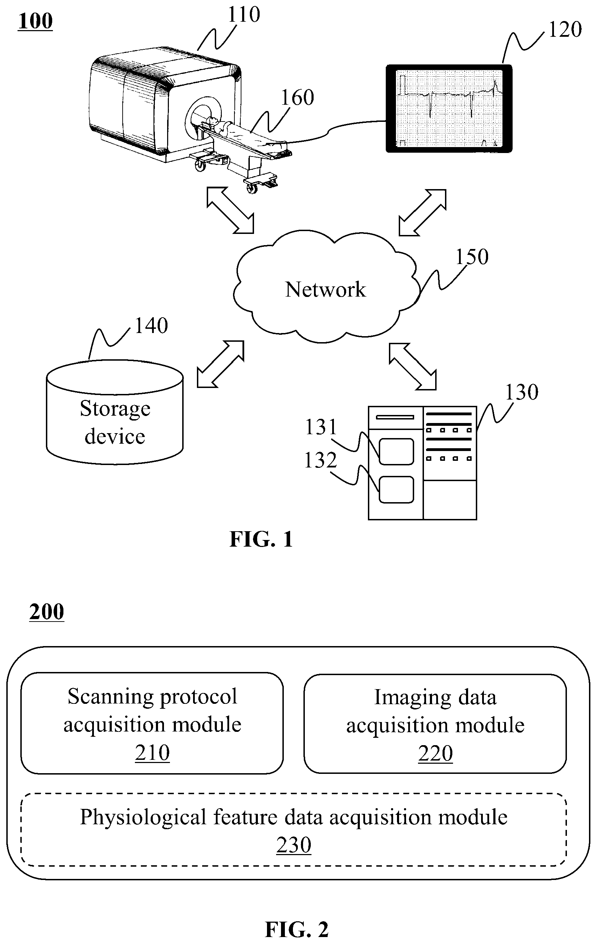

FIG. 1 is a schematic diagram illustrating an exemplary application scenario of an imaging system according to some embodiments of the present disclosure.

As shown in FIG. 1, the imaging system 100 may include a first device 110, a second device 120, a processing device 130, a storage device 140, and a network 150. In order to illustrate the practical application of the imaging system 100, the application scenario shown in FIG. 1 also includes an object 160.

The imaging system 100 may include one or more devices relating to medical imaging, and a technician may perform a medical imaging examination on the object 160 using the imaging system 100. The medical imaging examination may refer to a process of obtaining medical images of internal tissues of the object 160. For example, the imaging system 100 may include imaging devices and related devices using various imaging technologies such as magnetic resonance imaging (MR), positron emission tomography (PET), computed tomography (CT), ultrasound imaging, etc.

The imaging system 100 may obtain at least a portion of the imaging data relating to the object 160. The object may be a biological subject or a non-biological subject. For example, the object 160 may include a patient, an artificial subject, etc. As another example, the object 160 may include a specific part, an organ, and/or a tissue of a patient. For example, the object 160 may include the head, neck, chest, heart, stomach, blood vessels, soft tissues, tumors, nodules, or any combination thereof.

The first device 110 may be a main imaging device in medical imaging examination. For example, the first device 110 may include a PET device, a CT device, or a MR device. In some embodiments, the first device 110 may include a multi-modality imaging device. For example, the first device 110 may include a MR-PET device, a CT-PET device, etc.

In the medical imaging process, the object 160 may be placed at a data acquisition location (e.g., a scanning bed shown in FIG. 1) of the first device 110, and imaging data of the object 160 may be generated by the first device 110.

The second device 120 may be or include an auxiliary examination device in the medical imaging examination. In some embodiments, the second device 120 may be a physiological data detection device. In some embodiments, physiological feature data of the object 160 may be obtained using the second device 120 during the imaging process. In some embodiments, the second device 120 may contact with the object 160 through detection component(s) (e.g., a probe or a sensing patch) to obtain physiological feature data of the object 160.

In some embodiments, the second device 120 may include any other device that can assist the imaging process of the first device 110 and have data acquisition function(s). For example, the second device 120 may include a monitoring device, and the technician may determine whether a posture of the object 160 meets relevant imaging requirement(s) based on the monitoring device.

In some embodiments, the specific device(s) used for the first device 110 and/or the second device 120 may be determined based on specific inspection item(s) and related requirement(s). For example, for a coronary artery CT imaging examination, the first device 110 may be a CT device, and the second device 120 of the examination may include an electrocardiogram monitor used to obtain electrocardiogram data for assisting imaging and/or diagnosis. For example, the second device 120 in the coronary artery CT imaging examination may include a pulse tester to obtain pulse data of the object 160. The pulse data may be used to analyze whether the object 160 is in a tense state during imaging, thereby analyzing the reliability of the imaging data of the first device 110.

The processing device 130 may refer to a computing device (within the imaging system 100) used for imaging control and/or image data processing. In some embodiments, the processing device 130 may be implemented by a computing device with the data processing capability. For example, the processing device 130 may include a central processing unit (CPU), a graphics processing unit (GPU), a field programmable gate array (FPGA), etc.

In some embodiments, the processing device 130 may control the first device 110 and/or the second device 120 to execute the imaging methods provided in some embodiments of the present disclosure to obtain imaging data and/or physiological feature data of the object 160. In some embodiments, the processing device 130 may also perform methods for image data processing described in some embodiments of the present disclosure based on the received imaging data and/or physiological feature data, thereby achieving the associative storage of the imaging data and physiological feature data of the object 160.

It should be noted that the composition of the processing device 130 may not be limited in the present disclosure. In some embodiments, the processing device 130 may be a group of computing devices used in the imaging system 100. For example, the processing device 130 may include a computing device for controlling the imaging process (e.g., a control console of the first device 110 and/or the second device 120) and a computing device for data processing (e.g., an imaging data rendering device, a three-dimensional (3D) reconstruction device, etc.). In some embodiments, computing device(s) may be set up independently from the imaging system 100, and may be served as the processing device 130.

In some embodiments, as shown in FIG. 1, the processing device 130 may include an image data processing unit 131 and/or an imaging unit 132. The image data processing unit 131 may be used to execute the image data processing described in the present disclosure, and the imaging unit 132 may be used to execute imaging process(es) described in the present disclosure.

The storage device 140 may store data, instruction(s), and/or any other information. In some embodiments, the storage device 140 may store data obtained by the processing device 130. The data may include imaging data acquired from the first device 110 and/or physiological feature data acquired from the second device 120.

In some embodiments, the storage device 140 may be connected to the network 150 for communication with one or more other components (e.g., the first device 110, the second device 120, the processing device 130, etc.) of the imaging system 100. The one or more components of the imaging system 100 may access data or instructions stored in the storage device 140 via the network 150. In some embodiments, the storage device 140 may be a portion of the processing device 130.

The network 150 may include any suitable network that can facilitate information and/or data exchange of the imaging system 100. In some embodiments, one or more components of the first device 110 (e.g., a PET device), the second device 120, the processing device 130, the storage device 140, etc., may transmit information and/or data to one or more other components of the imaging system 100 via the network 150.

It should be noted that the above descriptions of the imaging system 100 is merely provided for illustrative purposes and may not be intended to limit the scope of the present disclosure. For those of ordinary skill in this field, various changes and modifications may be made under the guidance of the present disclosure. For example, the assembly and/or functionality of the imaging system 100 may be changed or altered according to specific embodiments. Merely by way of example, some other components, such as a power module that powers one or more components of the imaging system 100, other devices or modules, may be added to the imaging system 100.

FIG. 2 is a block diagram illustrating an exemplary imaging system according to some embodiments of the present disclosure.

As shown in FIG. 2, the imaging system 200 may include a scanning protocol acquisition module 210 and an imaging data acquisition module 220. In some embodiments, the imaging system 200 may be implemented on one or more processing devices 130.

In some embodiments, the scanning protocol acquisition module 210 may be configured to obtain scanning protocol data of an object. In some embodiments, the scanning protocol data may include an acquisition command relating to physiological feature data of the object, and the acquisition command may indicate to acquire or not to acquire the physiological feature data.

The imaging data acquisition module 220 may be configured to acquire imaging data of the object using a first device and based on the scanning protocol data.

The imaging system 200 may include a physiological feature data acquisition module 230.

The physiological feature data acquisition module 230 may be configured to obtain the physiological feature data of the object from a second device in response to that the acquisition command indicating to acquire the physiological feature data. In some embodiments, to obtain the physiological feature data of the object, the physiological feature data acquisition module 230 may be configured to obtain the physiological feature data from the second device at a beginning of an acquisition time period of the imaging data; and stop obtaining the physiological feature data from the second device at an end of the acquisition time period of the imaging data.

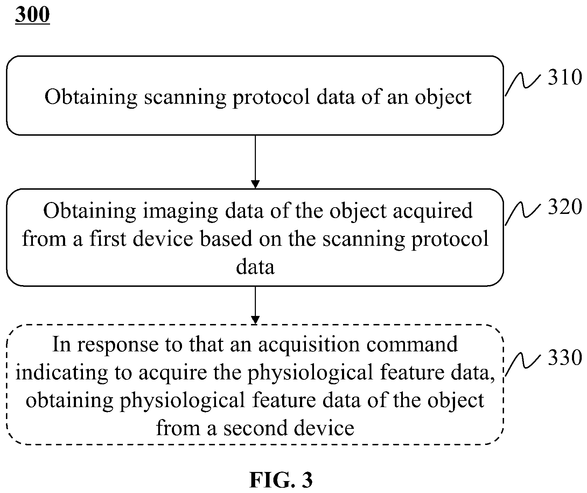

FIG. 3 is a flowchart illustrating an exemplary process of imaging according to some embodiments of the present disclosure. As shown in FIG. 3, the process 300 may include one or more of the following steps. In some embodiments, the process 300 may be performed by the imaging unit 132 or the imaging system 200.

In 310, scanning protocol data of an object (e.g., the object 160) may be obtained. In some embodiments, the operation 310 may be performed by the scanning protocol acquisition module 210.

A scanning protocol may refer to scanning requirement(s) of scanning device(s) (e.g., the first device and/or the second device) for a medical imaging examination. In some embodiments, the scanning protocol may be represented or expressed by the scanning protocol data. The scanning protocol data may be or include one or more scanning parameters in the medical imaging examination. For example, the scanning protocol data may include an imaging mode, working parameter(s) and relevant setting information of the scanning device(s).

In some embodiments, the technician may input one or more scanning parameter(s) on a preset page to determine at least a portion of the scanning protocol data of the object. For example, the technician may adjust an acquisition time period and/or an acquisition frequency on the preset page, and/or determine the scanning protocol data based on the adjusted scanning parameter(s).

The preset page may display scanning parameter(s) to be set in the imaging, and the technician may adjust a portion of the scanning protocol data on the preset page. For example, the technician may modify a numerical scanning parameter (e.g., the acquisition time period, the acquisition frequency, etc.) in the preset page. As another example, the technician may modify scanning parameter(s) (e.g., an imaging mode, a scanning region, etc.) by ticking corresponding option(s) on the preset page.

In some embodiments, the scanning protocol data may include scanning protocols corresponding to multiple examinations. For example, if a medical imaging examination of the object includes a lung CT examination and a coronary artery CT examination, and the examinations are to be executed by the same CT device, then the scanning protocol data may include a scanning protocol of the lung CT examination, a scanning protocol of the coronary artery CT examination, and an execution order of the examinations.

In some embodiments, the preset page may include multiple preset scanning protocols, and the technician may determine the scanning protocol data based on the preset scanning protocols. For example, the technician may determine one or more examinations to be performed on the object based on medical advice(s) of the object. For each examination, the technician may select a target scanning protocol from the preset scanning protocols and adaptively adjust scanning parameter(s) to generate scanning protocol data for the examination.

In 320, imaging data of the object acquired from a first device may be obtained based on the scanning protocol data. In some embodiments, operation 320 may be performed by the imaging data acquisition module 220.

The imaging data may refer to medical imaging data of the object generated or acquired by the first device directly. A data form of the imaging data may be related to the first device. For example, if the first device is an ultrasound device, the imaging data may be data of ultrasound image(s).

In some embodiments, the imaging data may be raw data that has not been used for image reconstruction. In some embodiments, the imaging data may be presented in a form of an image after being processed by one or more processing algorithms. For example, if the first device is a CT device, the imaging data may be X-ray beam projection data received by the X-ray beam receiver inside the CT device. By performing a data processing on the imaging data with a reconstruction algorithm (e.g., a Radon inverse conversion method), a CT image of the object may be obtained.

In some embodiments, in response to the scanning protocol data, one or more device parameters of the first device may be determined before a medical imaging examination is performed, and then the medical imaging examination may be performed at a corresponding acquisition time period to obtain the imaging data of the object.

In some embodiments, the acquisition time period may include a beginning time of the acquisition time period and an end time of the acquisition time period, and the first device may acquire the imaging data between the beginning time of the acquisition time period and the end time of the acquisition time period. In some embodiments, the beginning time of the acquisition time period may be determined by the technician, and the end time of the acquisition time period may be automatically determined based on an execution process of the medical imaging examination.

In some embodiments, the beginning time of the acquisition time period may also be determined based on physiological feature data acquired from a second device. For example, before the medical imaging examination, the physiological feature data may be acquired by the second device. If the physiological feature data meets data requirement(s) of acquiring the scanning protocol data, the medical imaging examination may be started, and this moment may be designated as the beginning time of the acquisition time period. For example, for the lung CT examination, the object may be requested to hold a breath, and the physiological feature data may be a respiratory signal. A corresponding data requirement may include that the lung CT examination is started in response to that the respiratory signal indicates the object is holding a breath.

In some embodiments, whether to obtain physiological feature data of the object from the second device while obtaining the imaging data may be determined based on actual requirement(s). The technician may determine whether to synchronously obtain the physiological feature data by ticking an acquisition command in setting or adjusting the scanning protocol data. If the acquisition command is selected, the second device may be directed to synchronously acquire the physiological feature data of the object, or the second device may be directed to synchronously transmit the physiological feature data of the object to the first device. If the acquisition command is not selected, the second device may not be directed to synchronously acquire the physiological feature data of the object, or the second device may not be directed to synchronously transmit the physiological feature data of the object to the first device.

In some embodiments, the acquisition command may be expressed by a scanning parameter with several options shown on the preset page. For example, the scanning parameter may include two option boxes indicating to acquire or not to acquire the physiological feature data. The technician may merely tick one of the two option boxes to indicate whether to synchronously acquire physiological feature data. As another example, the scanning parameter may include an acquisition option box. If the acquisition option box is ticked, the second device may be directed to synchronously acquire the physiological feature data of the object or transmit the physiological feature data to the first device. If the acquisition option box is not ticked, the second device may not be directed to synchronously acquire the physiological feature data of the object or not to transmit the physiological feature data to the first device. In some embodiments, the acquisition command may direct the second device to perform an off-screen acquisition of physiological feature data or direct the first device to obtain physiological feature data from the second device in an off-screen collection manner. The off-screen acquisition may refer to performing a data acquisition process in the background without displaying acquired data in real-time. The off-screen collection may refer to receiving the physiological feature data from the second device (e.g., a storage device of the second device) in the background . In some embodiments, the acquisition command may indicate real-time display of the acquired physiological feature data.

The process 300 may also include one or more of the following operations:
In 330, in response to that the acquisition command indicates to acquire the physiological feature data, the physiological feature data of the object may be obtained from a second device. In some embodiments, operation 330 may be performed by the physiological feature data acquisition module 230.

The physiological feature data may refer to data relating to physiological feature(s) of the object. The physiological feature data may be acquired from the second device and may be used for auxiliary imaging. In some embodiments, the physiological feature data may include at least one of a respiratory signal, an electrocardiogram signal, a pulse signal, a blood oxygen concentration, an electroencephalogram, an electromyography, a body temperature, a blood pressure, and/or an acousto-optic stimulation and a corresponding response, or any combination thereof. The specific content of the physiological feature data may be determined based on the actual requirements.

In some embodiments, the physiological feature data may be used to indicate a state of the object during the medical imaging examination. For example, the technician may determine whether the object is tense based on a pulse signal. As another example, the technician may determine whether the object is in a breath-holding state based on a respiratory signal.

In some embodiments, the physiological feature data may be used to assist to analyze the imaging data. For example, for a coronary artery CT imaging, the physiological feature data may include an electrocardiogram signal, a pulse signal, a blood oxygen concentration, and/or a blood pressure. Based on the physiological feature data, it is convenient for the technician to analyze the cardiovascular abnormalities of the object more comprehensively.

In some embodiments, in response to the acquisition command, the physiological feature data may be synchronously acquired during the acquisition time period of the imaging data. The physiological feature data synchronously acquired with the imaging data may be recorded as synchronous physiological feature data.

In some embodiments, an acquisition time period of the synchronous physiological feature data may be the same as the acquisition time period of the imaging data.

In some embodiments, the acquisition time period of the synchronous physiological feature data may be related to the acquisition time period of the imaging data. For example, the acquisition time period of the synchronous physiological feature data may be related to a relevant event occurs in the acquisition of the imaging data.

In some embodiments, the relevant event may occur in a time period beyond the acquisition time period of the imaging data. For example, when using the synchronous physiological feature data to guide the acquisition of the imaging data, the synchronous physiological feature data may be obtained first, and then the imaging data may be obtained when the synchronous physiological feature data meets a preset requirement (e.g., a heart rate of the object is within 100 beats/min). In some embodiments, the relevant event may occur within the acquisition time period of the imaging data. For example, the relevant event may include actions such as breath-holding and/or arm raising of the object.

In some embodiments, the acquisition time period of the synchronous physiological feature data may be manually determined by the user.

Based on the acquisition time period of the imaging data, the physiological feature data may be acquired from the second device at the beginning time of the acquisition time period of the imaging data, and the acquisition of the physiological feature data from the second device may be stopped at the end time of the acquisition time period of the imaging data. The physiological feature data acquired during the acquisition time period of the imaging data may be designated as the synchronous physiological feature data.

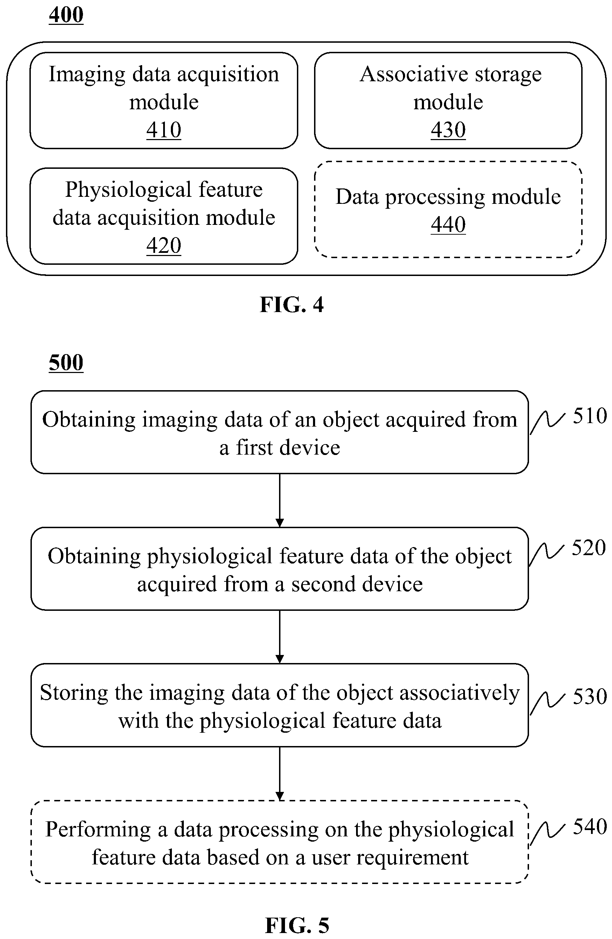

In some embodiments, the physiological feature data may be stored associatively with the imaging data of the object. More descriptions of the associative storage may be found elsewhere in the present disclosure, for example, operation 530 and the relevant descriptions in FIG. 5, which may not be repeated herein.

In some embodiments, the imaging data and the physiological feature data may be organized based on the scanning protocol data to determine the imaging data and the physiological feature data for each scanning protocol. In some embodiments, the physiological feature data corresponding to at least two scanning protocols may be arranged and displayed based on a presentation order of the imaging data corresponding to the at least two scanning protocols in the scanning protocol data.

FIG. 4 is a block diagram illustrating an exemplary system for image data processing according to some embodiments of the present disclosure.

As shown in FIG. 4, the system 400 for image data processing may include an imaging data acquisition module 410, a physiological feature data acquisition module 420, and an associative storage module 430.

The imaging data acquisition module 410 may be configured to obtain imaging data of an object acquired from a first device.

The physiological feature data acquisition module 420 may be configured to obtain physiological feature data of the object acquired from a second device. In some embodiments, the physiological feature data may at least include synchronous physiological feature data of the imaging data.

The associative storage module 430 may be configured to store the imaging data of the object associatively with the physiological feature data.

In some embodiments, as shown in FIG. 4, the system 400 may further include a data processing module 440. The data processing module 440 may perform a data processing operation on the physiological feature data based on user requirement(s). For example, the data processing module 440 may perform an image rendering operation on the physiological feature data to generate physiological feature image data. For example, the data processing module 440 may annotate, highlight a portion of the physiological feature data. More descriptions of further data processing of physiological feature data may be found in elsewhere in the present disclosure, for example, operation 540 and relevant descriptions, which may not be repeated herein.

It should be noted that the above descriptions of the system 400 for image data processing and its modules are only for the convenience of description, and cannot limit the present disclosure to the scope of the examples cited. It can be understood that for those skilled in the art, after understanding the principle of the system, it is possible to combine various modules arbitrarily, or form a subsystem to connect with other modules without departing from this principle. In some embodiments, the imaging data acquisition module 410, the physiological feature data acquisition module 420, the associative storage module 430, and the data processing module 440 shown in FIG. 4 may be different modules in one system, or one module may realize the functions of the above-mentioned two or more modules. For example, each module may share one storage module, or each module may have its own storage module. Variations such as these are within the scope of protection of the present disclosure.

FIG. 5 is a flowchart illustrating an exemplary process for image data processing according to some embodiments of the present disclosure. As shown in FIG. 5, the process 500 may include one or more of the following operations. In some embodiments, the process 500 may be performed by the image data processing unit 131 or the system 400.

In 510, imaging data of an object acquired from a first device is obtained. In some embodiments, operation 510 may be performed by the imaging data acquisition module 410.

In some embodiments, in operation 510, the imaging data acquired from the first device may be obtained from the first device or a background device associated with the first device. For example, the first device may acquire the imaging data according to the process 300 and the imaging data may be stored in the storage device 140. The imaging data in the storage device 140 may be directly obtained from the storage device 140 in the operation 510. As another example, the first device may send the imaging data to the image data processing system 400 while acquiring the imaging data.

More information of the imaging data may be found elsewhere in the present disclosure, for example, the operation 320 and the relevant descriptions, which may not be repeated herein.

In 520, physiological feature data of the object acquired from a second device is obtained. In some embodiments, operation 520 may be performed by the physiological feature data acquisition module 420.

In some embodiments, the physiological feature data at least includes synchronous physiological feature data of the imaging data acquired synchronously with the imaging data. For example, the second device may acquire the synchronous physiological feature data (i.e., the physiological feature data acquired through the off-screen acquisition) based on the process 300 and send the synchronous physiological feature data to the system 400. As another example, the second device may synchronously execute an acquisition operation on the physiological feature data (as described in operation 330) when the first device acquires the imaging data, and use the synchronously acquired physiological feature data as the synchronous physiological feature data.

In some embodiments, the physiological feature data may include physiological feature preparation data obtained before the beginning time of the acquisition time period of the imaging data. The physiological feature preparation data may be used to monitor whether the object meets imaging requirement(s). More descriptions of the physiological feature data may be found elsewhere in the present disclosure, for example, the operation 330 and the relevant descriptions, which may not be repeated herein.

In 530, the imaging data of the object is stored associatively with the physiological feature data. In some embodiments, operation 530 may be performed by the associative storage module 430.

The associative storage may refer to a storage of the physiological feature data and the imaging data based on a correlation between the physiological feature data and the imaging data. When reading the imaging data, the corresponding physiological feature data may be queried based on the correlation between the physiological feature data and the imaging data.

In some embodiments, the correlation between the physiological feature data and the imaging data may be determined based on an acquisition time. The physiological feature data and the imaging data with the same acquisition time or acquisition time period may be considered as corresponding data. For example, a medical imaging examination may include two examination items: a lung CT imaging and a coronary artery CT imaging, and the physiological feature data acquired during the acquisition time period of the examination items may correspond to the imaging data of the examination items.

In some embodiments, the imaging data may include a plurality of sub-sets of data according to the actual requirement(s). For example, each examination item may include at least two repeated scanning operations, and each scanning operation may include 120 sub-sets of X-ray beam projection data at different angles. Imaging data acquired in a medical imaging examination, an examination item, or a scanning operation, or the X-ray beam projection data may form a sub-set of data. In some embodiments, each sub-set of data may correspond to a set of physiological feature data based on the acquisition time period. For example, the physiological feature data corresponding to the X-ray beam projection data may be determined based on the acquisition time period of the X-ray beam projection data.

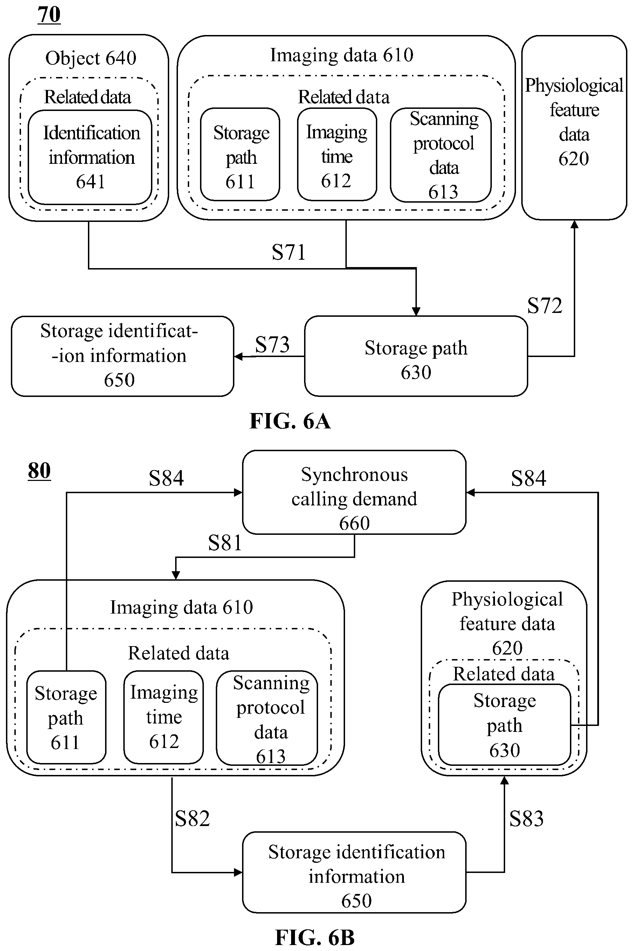

In some embodiments, a storage path of physiological feature data may be determined based on the correlation between the physiological feature data and the imaging data. More information of storing physiological feature data may be found elsewhere in the present disclosure, for example, FIG. 6 and the relevant descriptions, which may not be repeated herein.

In some embodiments, the process 500 may include a data processing operation 540 of the physiological feature data.

In 540, a data processing may be performed on the physiological feature data based on a user requirement. In some embodiments, operation 540 may be performed by the data processing module 440.

In some embodiments, the data processing may include rendering the physiological feature data. Physiological feature image data within a preset time period corresponding to the physiological feature data may be determined by rendering the physiological feature data within the preset time period. For example, for a pulse signal, the pulse signal may be presented on a two-dimensional (2D) image through rendering. The horizontal axis of the 2D image may represent the time, and the vertical axis of the 2D image may represent a count of pulses per minute. Since the pulse signal is detected by a periodic detection, the pulse signal may be presented as discrete points in the 2D image. The rendering operation of the pulse signal may be performed by sequentially connecting discrete points in the 2D image in chronological order to generate a pulse waveform. The pulse waveform may be used as the physiological feature image data.

In some embodiments, the physiological feature data may include one or more types of physiological signals, and the physiological feature image data may correspond to different sets of physiological feature image data corresponding to different types of physiological signals. In some embodiments, various physiological signals of the physiological feature data may be rendered based on an acquisition time and a chronological order indicated by the scanning protocol data, thus the physiological feature image data may be displayed in a chronological order.

In some embodiments, the rendering of the physiological feature data may be triggered by a rendering instruction of a user. For example, if the user inputs a rendering instruction, an image data processing device may respond the rendering instruction and generate the physiological feature image data of the object based on the physiological feature data of the object.

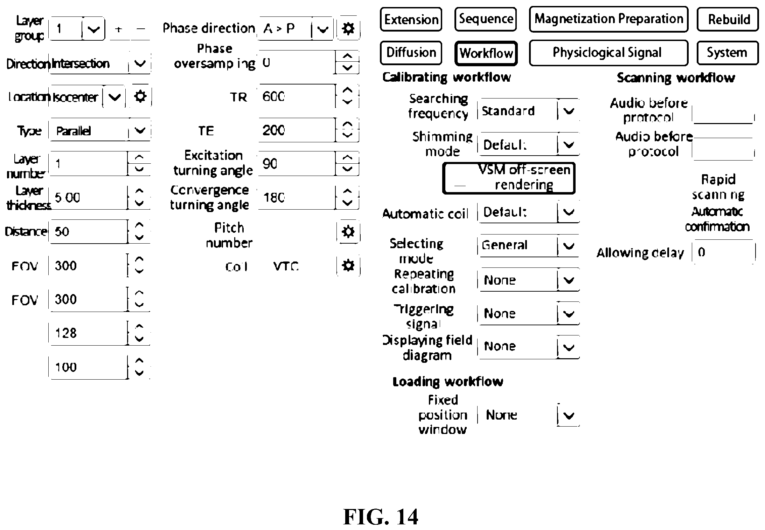

In some embodiments, a rendering selection controller may be generated for user interaction, and may be displayed on the preset page to enable the user to input the rendering instruction through the rendering selection controller. The rendering selection controller may be a visual interactive component. For example, the rendering selection controller may be a checkbox. When the user ticks the checkbox, it may be considered as inputting the rendering instruction. When the user does not tick the checkbox, it may be considered as not inputting the rendering instruction. More information of the rendering selection controller may be found elsewhere in the present disclosure, for example, FIG. 14 and the relevant descriptions.

In some embodiments, the physiological feature image data may be stored associatively with the imaging data of the object. The physiological feature image data may have a correlation with the object. More descriptions of the associative storage based on the correlation may be found elsewhere in the present disclosure, for example, the operation 530 and the relevant descriptions.

In some embodiments, in order to clarify the correlation between the physiological feature image data and the imaging data, when generating the physiological feature image data, the physiological feature data acquired in a time period may be determined based on a corresponding sub-set of the imaging data selected by the user, and the physiological feature data acquired in the corresponding time period may be rendered to determine the physiological feature image data.

In some embodiments, the imaging data and physiological feature image data of the object may be merged and displayed. For example, for a lung CT, each CT image may include the physiological feature image data acquired within the corresponding time period.

In some embodiments, the data processing on the physiological feature data may include an identification processing operation of the physiological feature data. The identification processing operation may be used to identify a specific or abnormal situation in the medical imaging examination, and analyze whether the corresponding imaging data is valid based on the identified physiological feature data.

In some embodiments, the technician may pre-determine the specific or abnormal situation based on scanning requirement(s). A preset specific or abnormal situation may be recorded as a target event, and a continuous examination may be conducted based on the target event in the medical imaging examination. For example, the second device may include a monitoring device, corresponding physiological feature data may include monitoring data, and the target event may include a motion of the object. If a severe motion is detected in the monitoring data, a duration time period of the severe motion may be recorded, and a corresponding monitoring video may be identified based on the duration time period to further analyze whether the motion affects the imaging quality. For example, for the purpose of examination, the first device or the technician may instruct the object to perform certain specific actions or present certain specific states, and the specific actions or the specific states may be designated as the target event. For example, a medical scanning device may indicate the object to perform an action through instruction(s) of "please hold your breath", "please raise your arm", etc. The target event may be a specific action or state that the object takes after receiving such instruction(s) from the medical scanning device.

In some embodiments, the data processing of the physiological feature data may further include performing a quality impact analysis on the imaging data of the object stored associatively with the physiological feature data. For example, an imaging quality of each set of imaging data may be scored based on a correlation between the physiological feature data and the imaging data, in order to determine the impact of changes of the physiological feature data on imaging quality. In some embodiments, the quality impact analysis may be performed on the imaging data based on the physiological feature image data.

To further illustrate the process of storing and calling the physiological feature data, FIGs. 6A and 6B of the present disclosure provide schematic diagrams of the process of storing and calling the physiological feature data. FIG. 6A is a schematic diagram illustrating an exemplary process for storing physiological feature data according to some embodiments of the present disclosure. FIG. 6B is a schematic diagram illustrating an exemplary process for calling physiological feature data according to some embodiments of the present disclosure.

As shown in FIGs. 6A and 6B, the process of storing and calling the physiological feature data involves imaging data 610, physiological feature data 620, and a storage path 630. The relevant descriptions of the imaging data 610, the physiological feature data 620, and the storage path 630 may be found elsewhere in the present disclosure, for example, the operation 530 and the relevant descriptions, which may not be repeated herein.

As shown in FIG. 6A, FIG. 6A is a schematic diagram illustrating an exemplary process for storing physiological feature data, which may include one or more of the following operations.

In S71, the storage path 630 of the physiological feature data 620 may be determined. The storage path 630 may be related to the imaging data 610.

As shown in FIG. 6A, the operation S71 may also involve an object 640. Related data of the imaging data 610 may include at least one of a storage path 611 of the imaging data, an imaging time 612 of the imaging data, and scanning protocol data 613 of the imaging data, or any combination thereof. Related data of the object 640 may at least include identification information 641 of the object.

The storage path 630 of the physiological feature data 620 may be determined during performing the operation S71 based on the related data of the imaging data 610 and/or the related data of the object 640.

The storage path 630 of physiological feature data 620 may be related to a file management mode of the imaging system. For example, when storing files, the imaging system may establish a primary address for each object based on the identification information 641 of the object, establish a secondary address based on date information of the imaging time 612 of the imaging data, and/or may establish a tertiary address based on a type of the physiological feature data 620. In some embodiments, the imaging system may generate the storage path 630 including multi-level addresses based on a specific content of the physiological feature data 620. For example, the storage path 611 may include a primary address, a secondary address, and a tertiary address. For pulse data acquired from a patient Peter on May 5, 2023, the storage path 611 may be represented as: Peter (primary address)/20230505 (secondary address)/pulse (tertiary address).

In S72, the physiological feature data 620 may be stored in the storage path 630.

In some embodiments, a physiological feature data file may be generated based on the physiological feature data 620 and may be stored in the corresponding storage path 630.

In some embodiments, for the convenience of querying the physiological feature data 620, the process 70 may further include one or more of the following operations.

In S73, storage identification information 650 may be generated based on the storage path 630 of the physiological feature data 620. The storage identification information 650 may be used to determine the correlation between the physiological feature data 620 and the imaging data 610.

In some embodiments, storage identification information 650 may be found through a physiological feature data pointer. The physiological feature data pointer may indicate the storage path 630 of the physiological feature data 620. For example, the physiological feature data pointer may include a count of bits of data and a specific position of the storage path 630 in a storage device. The physiological feature data 620 in the storage path 630 may be directly called based on the physiological feature data pointer.

In some embodiments, after determining that the physiological feature data 620 is stored in the storage path 630, the physiological feature data pointer indicating the storage path 630 of the physiological feature data 620 may be returned. The physiological feature data pointer may be filled into a specific data structure (e.g., a chain structure, a table structure) of the imaging data 610, thereby establishing the correlation between the physiological feature data 620 and the imaging data 610 through the physiological feature data pointer.

In some embodiments, the storage identification information 650 may include timestamp identification. When storing the imaging data and the physiological feature data file, the timestamp information may be generated based on specific time information of the imaging time 612 of the imaging data 610 and used as a title or other note information of the corresponding physiological feature data file, thereby establishing the correlation between the physiological feature data 620 and the imaging data 610 through the timestamp identification. For example, the title of the physiological feature data file may be 153812.153912, which may correspond to that the acquisition time period of the physiological feature data is from 15:38:12 to 15:39:12.

As shown in FIG. 6B, after storing the physiological feature data 620 based on the storage path 630 in the process 70, the storage path 630 of the physiological feature data 620 may be used as a portion of the related data of the physiological feature data 620.

When further processing the imaging data 610 and/or the physiological feature data 620, a synchronous calling demand 660 for the imaging data 610 and the physiological feature data 620 may be generated. To achieve a synchronous call of the imaging data 610 and the physiological feature data 620, the calling process in FIG. 6B may include one or more of the following operations.

In S81, in response to the synchronous calling demand 660, imaging data to be called may be determined.

The synchronous calling demand 660 may refer to a calling request that simultaneously calls the imaging data 610 and the physiological feature data 630. In some embodiments, the synchronous calling demand 660 may include active calling and/or passive calling. For example, if the technician is verifying a validity of the imaging data 610, the physiological feature data 620 may need to be analyzed, thus an active synchronous calling demand may be generated. As another example, when merging and displaying the imaging data 610 and the physiological feature data 620 (i.e., displaying a rendering result of the imaging data 610 and the physiological feature data 620 in a same medical image), the physiological feature data 620 acquired during the acquisition time period of the imaging data 610 may be displayed in a corresponding image, thus a passive synchronous calling demand may be generated.

In some embodiments, the imaging data 610 may be determined based on the synchronously calling demand 660. If the synchronous calling demand 660 needs to call a a portion of the imaging data 610 that is divided differently from the imaging data 610, the imaging data 610 may be re-divided based on the synchronous calling demand 660. For example, for the imaging data acquired from a CT device, X-ray projection data may be stored. If the synchronously calling demand 660 indicated to call a portion of the imaging data 610 corresponding to one or more scanning operations, the imaging data 610 including a plurality of sub-sets of X-ray projection data may be merged, and the corresponding physiological feature data of each X-ray projection data may be merged as the physiological feature data of the merged imaging data.

In S82, storage identification information 650 may be determined based on the imaging data 610.

In some embodiments, the storage identification information 650 of the imaging data 610 may be determined based on an identification mode of the storage identification information 650. A calling process of the storage identification information 650 may be a reverse process of generating the storage identification information 650.

In some embodiments, if the storage identification information 650 includes a physiological feature data pointer, the related data of the imaging data 610 may be recorded with a physiological feature data pointer, that is, the physiological feature data pointer may be directly called in the related data.

In some embodiments, if the storage identification information 650 includes a timestamp identification, the timestamp identification may be generated based on the imaging time 612 of the imaging data 610 and may be used as the storage identification information 650.

In S83, the physiological feature data 620 and the storage path 630 may be determined based on the storage identification information 650.

In some embodiments, the physiological feature data 620 corresponding to the object may be directly determined based on the storage identification information 650. For example, a storage address contained in the physiological feature data pointer may be directly accessed to achieve the calling of the physiological feature data 620. As another example, the timestamp identifications of files within a preset range (e.g., files with the same primary and secondary addresses) may be compared, and the physiological feature data with consistent timestamp identification may be designated as the physiological feature data corresponding to the imaging data.

In S84, a data calling may be performed based on the storage path 611 of the imaging data 610 and/or the storage path 630 of the physiological feature data 620.

In some embodiments, after confirming the storage path 611 of the imaging data 610 and/or the storage path 630 of the physiological feature data 620, a data accessing may be performed based on the storage path 611 and/or the storage path 630 in response to the synchronous calling demand 660, thereby calling the corresponding imaging data 610 and/or the physiological feature data 620.

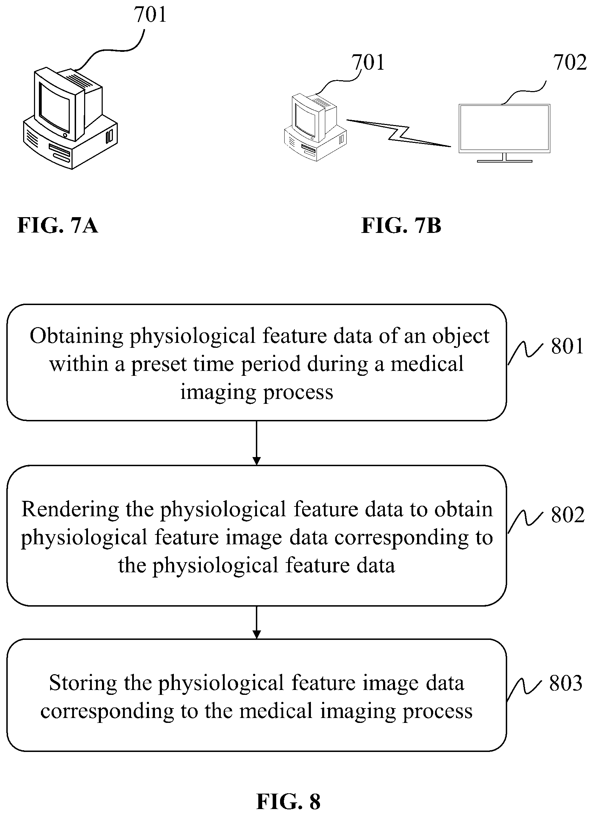

FIG. 7A is a schematic diagram illustrating an exemplary application scenario of a method for data processing according to some embodiments of the present disclosure. As shown in FIG. 7A, the application scenario may include a physiological feature data acquisition device 701 (e.g., the second device 120), which may be a specific acquisition device used to acquire the physiological feature data, such as electrocardiogram (ECG) data, electromyography (EMG) data, electroencephalogram (EEG) data, respiratory data, invasive blood pressure data, non-invasive blood pressure data, oxygen saturation data, end respiratory carbon dioxide data, body temperature data, cardiac output data, pulse data of the object, etc.

In the application scenario shown in FIG. 7A, during the medical imaging process, the physiological feature data acquisition device 701 may obtain physiological feature data of the object within a preset time period. The physiological feature data acquisition device 701 may render and process the physiological feature data to obtain physiological feature image data corresponding to the physiological feature data. The physiological feature image data may be displayed in a preset display form. The physiological feature data acquisition device 701 may store physiological feature image data corresponding to the medical imaging process, and the physiological feature image data may be used for quality impact analysis of medical images obtained from the medical imaging process.

Optionally, the application scenario involved in the data processing method provided in the embodiments of the present disclosure may further include a computing device 702 (e.g., a processing device 130) as shown in FIG. 7B, and communication between the physiological feature data acquisition device 701 and the computing device 702 may be carried out through a wired or wireless network. The computing device 702 may be an intelligent robot, a smartphone, a tablet, a personal computer, a laptop, a wearable device, a server, etc.

In the application scenario shown in FIG. 7B, during the medical imaging process, the physiological feature data acquisition device 701 may obtain the physiological feature data of the object within a preset time period, and send the physiological feature data to the computing device 702. After the computing device 702 obtains the physiological feature data, the physiological feature data may be rendered and processed to obtain the corresponding physiological feature image data. The physiological feature image data may be displayed in a preset displaying format to visualize the physiological feature data. The computing device 702 may store physiological feature image data corresponding to the medical imaging process. The physiological feature image data may be used for quality impact analysis of a medical image acquired in the medical imaging process.

In some embodiments, as shown in FIG. 8, a data processing method may be performed on the computing device 702 shown in FIG. 7B, which may include one or more of the following operations.

In 801, physiological feature data of an object within a preset time period during a medical imaging process may be obtained by a computing device.

The medical imaging process may include magnetic resonance imaging, computed tomography (CT) imaging, positron emission tomography (PET) imaging, and ultrasonic (US) imaging, or the like, or a combination thereof. In some embodiments, the medical imaging may involve a long-term or continuous imaging, such as magnetic resonance imaging, multiple CT imaging, PET imaging, intraoperative angiography, etc.

In the medical imaging process of the object, for example, in the medical imaging process of an abdomen, a chest, a head, or other parts of the object, a medical scanning device may be used to scan the object to obtain medical image(s), and the computing device may obtain physiological feature data of the object within the preset time period.

The preset time period may be a time period between a beginning of scanning and an end of the scanning corresponding to a scanning protocol of the object. Different body parts may correspond to different scanning protocols. The scanning protocol may be a set of scanning parameters used by a medical scanning device to scan the medical images of a corresponding body part of the object based on the scanning protocol.

It should be noted that the preset time period may also be greater than or less than the time period between the beginning of scanning and the end of the scanning mentioned above. For example, if the preset time period is less than the time period between the beginning of scanning and the end of the scanning mentioned above, the preset time period may be an event period during a process of the beginning of scanning and the end of the scanning where the event occurs, such as an event of breath holding or arm raising of the object. Optionally, the preset time period may also be a time period manually set by the user.

The preset time period may be taken as an example to illustrate the time period between the beginning of scanning and the end of the scanning corresponding to a scanning protocol.

As mentioned above, the physiological feature data of the object may be obtained by the physiological feature data acquisition device during the preset time period. The physiological feature data may at least include the ECG data, the EMG data, EEG data, respiratory data, invasive blood pressure data, non-invasive blood pressure data, oxygen saturation data, end respiratory carbon dioxide data, body temperature data, cardiac output data, pulse data of the object.

After the physiological feature data acquisition device obtains the physiological feature data of the object within the preset time period, the physiological feature data may be sent to the computing device. In this way, the computing device may obtain the physiological feature data of the object within the preset time period.

In 802, the physiological feature data may be rendered by the computing device to obtain physiological feature image data corresponding to the physiological feature data.

The physiological feature image data may be displayed in a preset displaying format to visualize the physiological feature data.

In some embodiments, the physiological feature data may include feature data corresponding to multiple sampling moments within the preset time period. For example, if the physiological feature data acquisition device obtains feature data once per second within the preset time period, the multiple sampling moments may be the 1st second, the 2nd second, and so on within the preset time period.

In this way, the computing device may draw feature points in a preset coordinate system based on each sampling moment and the corresponding feature data at the sampling moment, and obtain the physiological feature image data based on the feature points.

Taking the respiratory data of the object as an example, the X-axis in the preset coordinate system may represent the sampling moments, and the Y-axis may represent the respiratory data (or normalized respiratory data). A position of each feature point in the preset coordinate system may be determined by a corresponding sampling moment and respiratory data. For example, a coordinate on the Y-axis of a feature point corresponding to a moment of the 45-th second in the X-axis may be the respiratory data of the object acquired at the 45-th second. It should be noted that, as mentioned above, in the present disclosure, the preset time period is used as an example of the time period between the beginning of scanning and the end of the scanning corresponding to a scanning protocol, and the above sampling moments can be any moment between the beginning of scanning and the end of the scanning.

In some embodiment, the physiological feature image data may be displayed based on the feature points and preset coordinate system, that is, the physiological feature image data may be displayed in the form of feature points.

In some embodiments, the physiological feature image data may be displayed in the preset coordinate system by connecting the feature points arranged in the chronological order of the sampling moments in a broken line. In this way, the physiological feature image data may be displayed in a broken line format. For example, the respiratory cycle of the object may be reflected in the physiological feature image data, specifically, two adjacent peaks and valleys in the physiological feature image data may be designated as a respiratory cycle of the object.

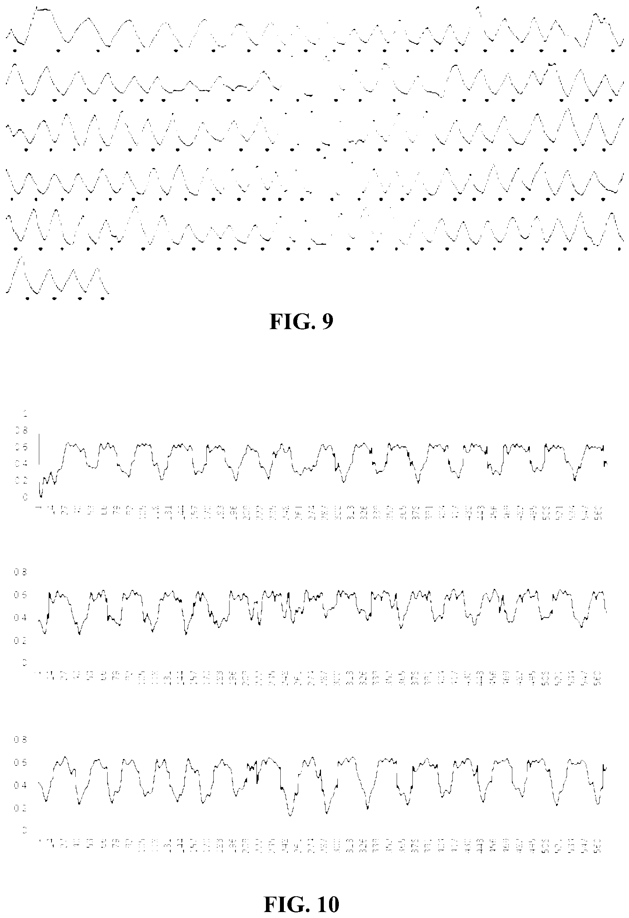

Referring to FIG. 9, FIG. 9 is a schematic diagram illustrating exemplary physiological feature image data representing respiratory data of an object according to some embodiments of the present disclosure. As shown in FIG. 9, the curve lines may be fitted from the respiratory data of the object. As an example, the computing device may add respiratory cycle data corresponding to respiratory data to the physiological feature image data. The respiratory cycle data may be shown at the points below the curve lines in FIG. 9, where each point may represent a moment, and the time period between two adjacent points may be a respiratory cycle (also referred to as a heartbeat cycle), to facilitate a more intuitive analysis of the respiratory data within each heartbeat cycle.

Furthermore, the computing device may display a connection of feature points in the physiological feature image data in rows. For example, each row may display data in a fixed duration (e.g., 1 minute), and if the fixed duration is exceeded, it may be switched to the next row until the scanning may be completed, thereby ensuring the aesthetics and continuity of the physiological feature image data.

Referring to FIG. 10, FIG. 10 is another schematic diagram illustrating exemplary physiological feature image data representing respiratory data of an object according to some embodiments of the present disclosure. As shown in FIG. 10, the computing device may display the respiratory data in 560 seconds per row in the physiological feature image data, and switch to the next row to continue displaying if the duration exceeds 560 seconds, thereby ensuring the aesthetics and data continuity of the physiological feature image data.

In some embodiments, the preset displaying form may not be limited to the line chart of the above example, but also may be a bar chart, a pie chart, a discrete chart, or other forms, which may not be limited herein.

In some embodiments of the present disclosure, the computing device may call an off-screen rendering controller to render the physiological feature data. For example, the computing device may bind the physiological feature data to the off-screen rendering controller. The binding operation may be understood as designating the physiological feature data as input data of the off-screen rendering controller, thereby rendering the physiological feature data using the off-screen rendering controller.

The off-screen rendering controller may start to render the physiological feature data based on a preset image size and/or a preset rendering order. The rendering order may be a chronological order. For example, during the rendering process, the off-screen rendering controller may periodically detect whether an image with the preset image size may be fully rendered. If the image is fully rendered, the currently rendered data may correspond to an image, and the image may be stored in memory before the current scan may be completed.

Similarly, after the current scan is completed, the computing device may merge multiple images stored in memory into one image to obtain the physiological feature image data mentioned above. The computing device may store the physiological feature image data and/or clear the multiple images stored in memory.

As mentioned above, if the computing device merges multiple images stored in memory into one image, feature points may be connected in the chronological order of the sampling moments to form a broken line, and the broken line may be displayed in the preset coordinate system. The image of the physiological feature image data may be in any format, which may not be specifically limited in the embodiments of the present disclosure.

As an example, the off-screen rendering controller may periodically detect whether the image with the present size is fully rendered during the drawing process. If the image is fully rendered, a full-rendering flag bit may be set. If the computing device detects the full-rendering flag bit, the currently rendered data may correspond to an image.

Afterwards, the computing device may also clear the physiological feature data bound to the off-screen rendering controller, so that the off-screen rendering controller may bind new physiological feature data for rendering.

In 803, the physiological feature image data corresponding to the medical imaging process may be stored by the computing device.

Optionally, the computing device may store the physiological feature image data in a designated directory corresponding to the medical imaging process.

As an example, the computing device may store the physiological feature data of the object within the preset time period in the directory, which may include original physiological feature data and corresponding physiological feature image data stored by the computing device. Therefore, when the computing device needs to use the physiological feature image data corresponding to the physiological feature data, the physiological feature image data may be obtained directly from the directory without the need of rendering. In addition, the physiological feature image data may be rendered by a rendering indication parameter stored in the scanning protocol data, which may be beneficial for improving the control flexibility of shading processing.

It should be noted that in the embodiments of the present disclosure, the directory may be a storage directory on a hard disk of the computing device, which may avoid the problem of data loss caused by storing data in memory and improve the data security of physiological feature data and corresponding physiological feature image data.

Optionally, the computing device may store the physiological feature image data corresponding to the medical image, the identity of the object (e.g., a name, a telephone number or an ID card number of the object), the acquisition date of the medical image, and/or the scanning protocol data corresponding to the medical image at the preset storage location. In this way, by associating the physiological feature image data with the object, the traceability is improved, which may be beneficial to track the physiological feature data of a certain scanning protocol for the object.

This physiological feature image data may be used to analyze a quality impact of a medical image obtained during the medical imaging process, such as analyzing a physiological change of the object during the medical imaging process, whether the physiological change of the object is the reason for an unclear image, and which specific physiological change may cause the unclear image. In some embodiments, the physiological feature data may be segmented based on the object and/or scanning protocol, that is, the physiological feature image data of different objects and/or scanning protocols may be stored separately, which may accurately reflect the physiological feature data of a certain scanning protocol of the object, facilitating the analysis of the relationship between the physiological feature data and the medical image.

In some embodiments, the computing device may independently display the physiological feature image data and the medical image corresponding to the aforementioned medical imaging process.

In some embodiments, the computing device may obtain a plurality of medical images corresponding to the medical imaging process and merge them with the physiological feature image data for displaying. For example, a medical image and the physiological feature image data may be displayed separately in different regions of an image, which may avoid time waste of user(s) searching for medical image(s) and physiological feature image data separately. Merging the medical image(s) with the physiological feature image data for displaying may facilitate user(s) to visually compare the medical image(s) with the physiological feature image data, making it more convenient to analyze the impact of the physiological change of the object on the imaging quality during the medical imaging process.

It should be noted that physiological feature data may include two or more of the ECG data, the EMG data, EEG data, respiratory data, invasive blood pressure data, non-invasive blood pressure data, oxygen saturation data, end respiratory carbon dioxide data, body temperature data, cardiac output data, and pulse data of the object, or the like, or a combination thereof. For different physiological feature data, the computing device may generate different physiological feature image data corresponding to the physiological feature data, which may not be limited herein.

In some embodiments, if the physiological feature data acquisition device is abnormal, such as loosening or being removed, the computing device may prohibit the performing process of operations 802 and 803, that is, no further rendering processing may be performed on the physiological feature data, and no physiological feature image data may be stored, thus to prevent interference from abnormal signals and reduce unnecessary computation.

The physiological feature data of the object within the preset time period in the above embodiments may be obtained during the medical imaging process, and then the physiological feature data may be rendered to obtain physiological feature image data corresponding to the physiological feature data. The physiological feature image data may be used to express the physiological feature data in a preset displaying form, and then the physiological feature image data may be stored corresponding to the medical imaging process. The physiological feature image data may be used for quality impact analysis of medical image(s) acquired in the medical imaging process. In this way, during the medical imaging process, the physiological change of the object may have a direct impact on the imaging quality of the medical image(s). For example, during a vascular imaging, if the object is too nervous and flustered, the blood may flow faster, thereby affecting the imaging quality. During neuroimaging, if the nervous system of the object is tense due to mental tension, the imaging quality may be affected. In view of this, the physiological feature image data obtained by rendering the physiological feature data of the object in the medical imaging process may be stored, through which the impact of the physiological change(s) of the object on the imaging quality of the corresponding medical imaging process may be effectively analyzed. In some embodiments, whether the physiological change of the object is the cause for unclear image(s), and which specific physiological change may cause the unclear image(s) may be analyzed, which is beneficial to improve the quality of medical images based on an analysis result.

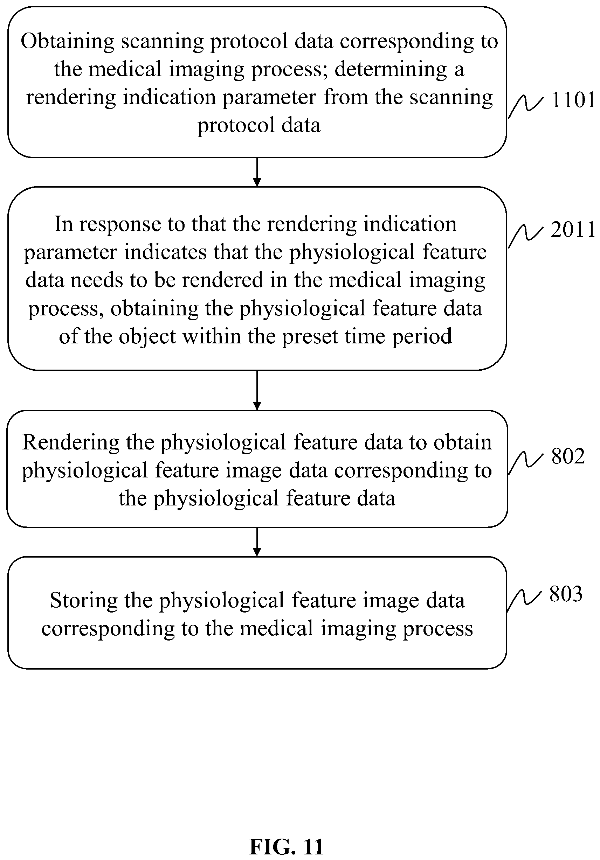

In some embodiments, based on the embodiments shown in FIG. 8, as shown in FIG. 11, the process may relate to the timing of obtaining the physiological feature data by the computing device. As shown in FIG. 11, the data processing process may further include operation 1101.

In 1101, scanning protocol data corresponding to the medical imaging process may be obtained, and a rendering indication parameter may be determined from the scanning protocol data.

In some embodiments of the present disclosure, the rendering indication parameter may be pre-bound with the scanning parameter(s) corresponding to the scanning protocol to obtain scanning protocol data. In this way, the scanning protocol data may be used to control whether the rendering processing is required to be performed on the physiological feature data during the scanning process based on the scanning protocol, that is, whether the rendering processing is triggered to perform on the physiological feature data may be determined based on the rendering indication parameter.

In some embodiments, the computing device may automatically perform the rendering processing on the physiological feature data, or may not perform the rendering processing on the physiological feature data based on the rendering indication parameter in the scanning protocol data.

Correspondingly, the computing device may perform the following operation 2011 to achieve operation 801 mentioned-above.

In 2011, in response to that the rendering indication parameter indicates that the physiological feature data needs to be rendered in the medical imaging process, operations for obtaining the physiological feature data of the object within the preset time period may be performed by the computing device.

In this way, if the rendering indication parameter indicates that the physiological feature data needs to be rendered in the medical imaging process, the computing device may perform the operation for obtaining the physiological feature data of the object within the preset time period, render the physiological feature data to obtain the physiological feature image data corresponding to the physiological feature data, and/or store the physiological feature image data corresponding to the medical imaging process. Thus, the user may view the physiological feature image data directly at a corresponding storage address, and the physiological feature data may also be stored correspondingly for the convenience of the user viewing.

Based on the embodiments shown in FIG. 8 or FIG. 11 above, in some embodiments, the data processing of the present disclosure may further include one or more of operations A1 and A2.

In A1, the rendering indication parameter corresponding to the medical imaging process may be obtained by the computing device.

The rendering indication parameter may be used to indicate whether the physiological feature data needs to be rendered in the medical imaging process. The rendering indication parameter may be configured and input by the user.

In A2, the computing device may store the rendering instruction parameter as a protocol parameter corresponding to the medical imaging process in the scanning protocol data corresponding to the medical imaging process.

The computing device may use the rendering instruction parameter as a protocol parameter corresponding to the medical imaging process, bind them with the scanning parameter(s) of the scanning protocol corresponding to the medical imaging process to obtain the scanning protocol data and store the scanning protocol data.

The rendering indication parameter may be bound to the scanning parameter(s) corresponding to the scanning protocol. In some embodiments, the computing device may automatically render or may not render the physiological feature data based on the instruction of the rendering indication parameter in the scanning protocol data. For each scanning protocol, implementing the control of whether to trigger the rendering of physiological feature data based on the rendering indication parameter, and controlling whether to render the physiological feature data may be controlled by a parameter of the scanning protocol, thus improving the convenience and flexibility of the rendering processing performed on the physiological feature data.

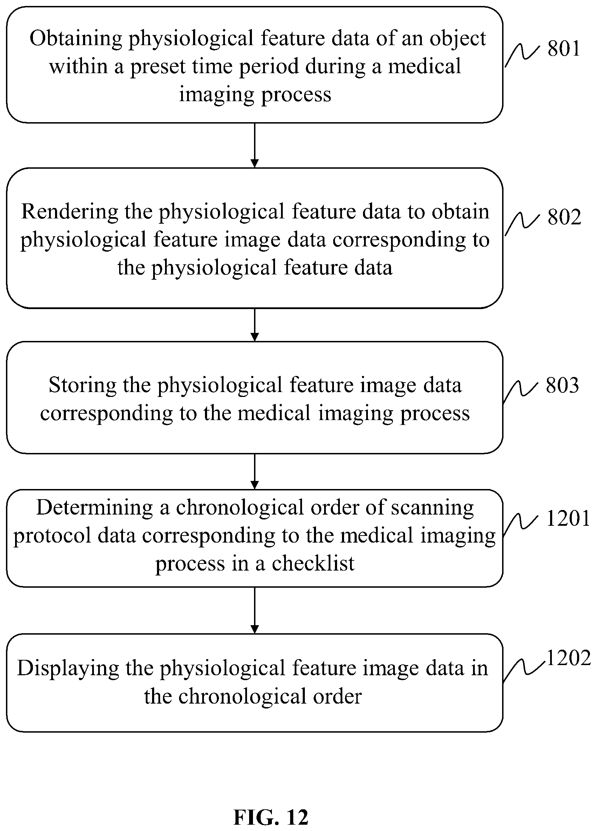

In some embodiments, based on the embodiment shown in FIG. 8, as shown in FIG. 12, which relates to a process of displaying the physiological feature image data by the computing device, the data processing may further include one or more of operations 1201 and 1202.

In 1201, a chronological order of scanning protocol data corresponding to the medical imaging process in a checklist may be determined by the computing device.

The checklist may be a list of examination sequence(s) corresponding to different examination items for the object. For example, a first piece of scanning protocol data in the checklist may be scanning protocol data corresponding to an abdominal examination item, and a second piece of scanning protocol data in the checklist may be scanning protocol data corresponding to a chest examination item, which may indicate that the object may be first scanned for the abdomen, and then scanned for the chest.

Optionally, the checklist may be a list of examination sequence(s) of the same examination item for different objects. For example, if the examination item is for an abdominal examination, the first piece of scanning protocol data in the checklist may be the scanning protocol data of the object A, and the second piece of scanning protocol data in the checklist may be the scanning protocol data of the object B, which may indicate that the abdominal scan may be first performed on the object A, and may be next performed on the object B.

In 1202, the physiological feature image data in the chronological order may be displayed by the computing device.

In this way, the computing device may display the physiological feature image data in the chronological order according to the scanning protocol data in the checklist.

Optionally, the medical imaging process, i.e.. one or more examination items, may be carried out in the chronological order, and the computing device may display the physiological feature image data of the current medical imaging process after each medical imaging process is completed.

Optionally, the computing device may display the physiological feature image data in the chronological order after obtaining the corresponding physiological feature image data for each medical imaging process.

Optionally, after obtaining the physiological feature image data corresponding to each medical imaging process, the computing device may display the physiological feature image data in the chronological order upon receiving a display instruction from the user.

The chronological order of the scanning protocol data corresponding to the medical imaging process in the checklist may be determined in above embodiments, and the physiological feature image data may be displayed in the chronological order, which may facilitate the analysis of patients' emotional fluctuations during the scanning process and the impact of the patients' emotional fluctuations on the quality of the collected images in order of examination, and may be conducive to improving analysis efficiency.

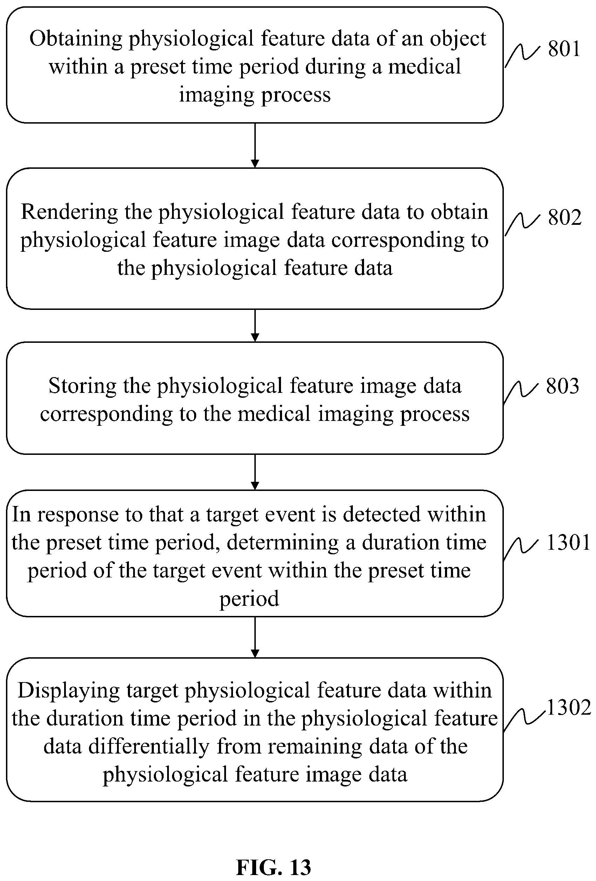

Based on the embodiment shown in FIG. 8, as shown in FIG. 13, which involves a process of differentially displaying the physiological feature data of a target event by the computing device, the data processing may further include one or more of operations 1301 and 1302.

In 1301, in response to that a target event is detected within the preset time period, a duration time period of the target event within the preset time period is determined by the computing device.

In the medical imaging process, for the purpose of examination, the medical scanning device may instruct the object to perform certain specific actions or present certain specific states. For example, the medical scanning device may indicate the object to perform actions using instructions of "please hold your breath", "please raise your arm", etc. The target event may be a specific action or a specific state of the object after receiving an instruction from the medical scanning device.

In some embodiments of the present disclosure, the computing device may communicate with the medical scanning device via a wired or wireless network. The medical scanning device may instruct the object to perform the target event, and/or may send a target event start reminder and/or a target event end reminder to the computing device. In this way, the computing device may determine the duration time period of the target event based on a receiving moment of the target event start reminder and a receiving moment of the target event end reminder.

In 1302, target physiological feature data within the duration time period in the physiological feature data is displayed (by the computing device) differentially from remaining data of the physiological feature image data.

The differential display may be achieved by subtracting the target physiological feature data from the physiological feature image data, or highlighting the target physiological feature data in a color that is different from the remaining physiological feature data.

In this way, by differentially displaying the target physiological feature data corresponding to the target event in the physiological feature image data, the user may be prominently reminded of the target event during the medical imaging process, avoiding the user from ignoring the target event. Analyzing the impact of the physiological change in the object on the imaging quality during the corresponding medical imaging process may be beneficial to improve the accuracy of the analysis.

In some embodiments, the computing device may further display a rendering selection controller, which may be used for the user to choose whether to render the physiological feature data.

For example, as shown in FIG. 14, which is a schematic diagram illustrating an exemplary interface of a rendering selection controller according to some embodiments of the present disclosure. The computing device may display the rendering selection controller for the user to choose whether to render the physiological feature data. For example, if the user selects "VSM(Vital Signs Monitor) off-screen rendering", it may indicate that the user has chosen to render the physiological feature data.

In this way, if the computing device detects a confirmation instruction input by the user through the rendering selection controller, the computing device may render the physiological feature data to obtain the corresponding physiological feature image data.

In some embodiments, an interface shown in FIG. 14 may be designated as the preset page mentioned above. A wording "VSM off-screen rendering" and a box before the wording shown in FIG. 14 may be the rendering selection controller. In some embodiments, the scanning protocol data and the acquisition command may be determined based on an interactive controller (e.g., an acquisition selection controller) on the preset page. For example, similar to the rendering selection controller, an acquisition selection controller may be built to facilitate the input of the acquisition command. Whether to acquire the synchronous physiological feature data may be determined based on the user's selection of a checkbox that represents the acquisition selection controller.

In some embodiments of the present disclosure, in addition to binding the rendering indication parameter with the scanning parameter(s) corresponding to the scanning protocol in advance, and controlling whether to trigger the rendering processing on the physiological feature data based on the rendering indication parameter, the rendering selection controller may be displayed for the user to manually choose whether to render the physiological feature data, improving the flexibility of rendering the physiological feature data.

It should be understood that although the various operations in the flowchart related to the embodiments mentioned above may be displayed in sequence as indicated by the arrows, these operations may not be necessarily executed in the order indicated by the arrows. Unless explicitly stated in this article, there may be no strict order limit for the execution of these operations, and these operations may be executed in other order. Moreover, at least a portion of the operations in the flowchart involved in the various embodiments mentioned above may include multiple steps or stages, which may not necessarily be completed at the same time, but may be executed at different times, and the execution order of these steps or stages may not necessarily be sequential. Instead, it may be executed alternately or alternately with other operations or at least a portion of steps or stages within other operations.

Based on the same inventive concept, the present embodiment also provides a data processing device for implementing the aforementioned data processing method. The implementation scheme provided by the device to solve the problem may be similar to the implementation scheme recorded in the above method. Therefore, the specific limitations in one or more data processing device embodiments provided below may be referred to in the previous text for the limitations of data processing methods, and may not be repeated herein.

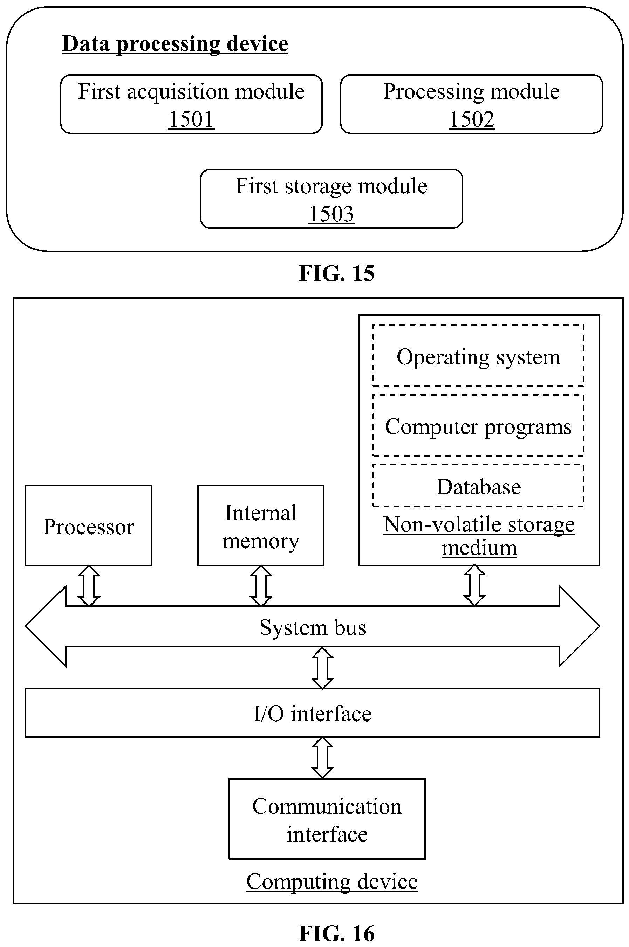

In some embodiments, as shown in FIG. 15, a data processing device is provided. The device may include one or more of the following components.

A first acquisition module 1501 may be configured to obtain physiological feature data of the object within a preset time period during a medical imaging process.

A processing module 1502 may be configured to render the physiological feature data to obtain physiological feature image data corresponding to the physiological feature data. The physiological feature image data may be used to express the physiological feature data in a preset displaying format.

A first storage module 1503 may be configure to store the physiological feature image data corresponding to the medical imaging process, and the physiological feature image data may be configured to be used for quality impact analysis of a medical image acquired in the medical imaging process.

In some embodiments, the device may further include one or more of the following components.

A second acquisition module may be configured to obtain scanning protocol data corresponding to the medical imaging process and determine a rendering indication parameter from the scanning protocol data.

The first acquisition module 1501 may specifically be used to perform operations for obtaining the physiological feature data of the object within the preset time period in response to that the rendering indication parameter indicates that the physiological feature data needs to be rendered in the medical imaging process.

In some embodiments, the device may further include one or more of the following components.

A third acquisition module may be configured to obtain a rendering indication parameter corresponding to the medical imaging process, and the rendering indication parameter may be configured to indicate whether the physiological feature data needs to be rendered in the medical imaging process.

A second storage module may be configured to store the rendering indication parameter as a protocol parameter corresponding to the medical imaging process in scanning protocol data corresponding to the medical imaging process.

In some embodiments, the device may further include one or more of the following components.

A fourth acquisition module may be configured to obtain the medical image corresponding to the medical imaging process.

A first displaying module may be configured to display the medical image associatively with the physiological feature image data.

In some embodiments, the device may further include one or more of the following components.

A first determination module may be configured to determine a chronological order of scanning protocol data corresponding to the medical imaging process in a checklist.

A first showing module may be configured to display the physiological feature image data in the chronological order.

In some embodiments, the device may further include one or more of the following components.

A second determination module may be configured to determine a duration time period of the target event within the preset time period in response to that a target event may be detected within the preset time period.

A second displaying module may be configured to display target physiological feature data within the duration time period in the physiological feature data differentially from remaining data of the physiological feature image data.

In some embodiments, the device may further include one or more of the following components.

A second showing module may be configured to display a rendering selection controller, and the rendering selection controller may be used for a user to choose whether to render the physiological feature data.

The processing module 1502 may be used to render the physiological feature data and obtain the corresponding physiological feature image data in response to that a confirmation instruction input by the user is detected based on the rendering selection control.

In some embodiments, the first storage module 1503 may be used to store the physiological feature image data corresponding to the medical image, an identification of the object, an acquisition time of the medical image, and scanning protocol data corresponding to the medical image at a preset storage location.

The various modules in the above data processing device may be fully or partially implemented through software, hardware, or their combinations. The above modules may be embedded in or independent from a processor in a computing device in hardware form, or may be stored in memory in the computing device in software form for the processor to call and execute the corresponding operations of the above modules.

In some embodiments, a computing device may be provided, which may include a storage device, a display device, and a processor. The storage device may store computer programs, and the processor may execute the computer programs to: obtain physiological feature data of an object within a preset time period, the physiological feature data being obtained in real-time during a medical imaging process, and the medical imaging process being obtained during a historical time period; render the physiological feature data to obtain physiological feature image data corresponding to the physiological feature data, wherein the physiological feature image data may be configured to be used for quality impact analysis of a medical image corresponding to the historical time period; and/or control the display device to display the physiological feature image data and/or the medical image corresponding to the historical time period.

The operations and beneficial effects that the processor may implement when executing computer programs may be found the limitations on data processing methods in the previous text, which may not be repeated herein.

In some embodiments, a computing device may be provided, which may be a server. The internal structure of the computing device may be shown in FIG. 16. The computing device may include a processor, a memory, and a network interface connected by a system bus. The processor of the computing device may be used to provide computing and control capabilities. The memory of the computing device may include a non-volatile storage medium and an internal memory. This non-volatile storage medium may store an operating system, computer programs, and a database. The memory may provide an environment for the operations of operating systems and computer programs on non-volatile storage medium. The database of the computing device may be configured to store data processing data. The network interface of the computing device may be configured to communicate with external terminals via a network connection. The computer programs may be executed by the processor to implement a data processing method.

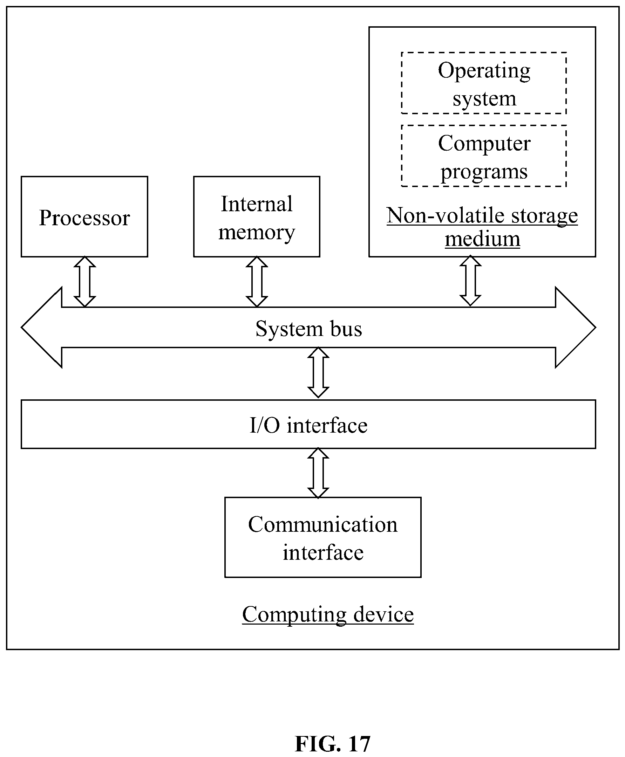

In some embodiments, a computing device may be provided, which may be a terminal. The internal structure of the computing device may be shown in FIG. 17. The computing device may include a processor, a memory, a communication interface, a displaying screen, and an input device connected by a system bus. The processor of the computing device may be used to provide computing and control capabilities. The memory of the computing device may include a non-volatile storage medium and an internal memory. The non-volatile storage medium may store an operating system and computer programs. The memory may provide an environment for the operations of the operating system and computer programs on the non-volatile storage medium. The communication interface of the computing device may be used for wired or wireless communication with external terminals, which may be achieved by WIFI, mobile cellular networks, near field communication (NFC), or other technologies. The computer programs may be executed by the processor to implement a data processing method. The displaying screen of the computing device may be a liquid crystal displaying screen or an electronic ink displaying screen. The input device of the computing device be a touch layer covered by the displaying screen, or a key, a trackball or a touch pad set on the computing device shell, or an external keyboard, a touch pad or a mouse.

Those skilled in the art may understand that the structures shown in FIGs. 16 and 17 are merely schematic diagrams of a portion of the structures related to the present disclosure, and may not constitute a limitation on the computing device. A specific computing device may include more or fewer components than shown in FIGs. 16 and 17, or combinations of certain components, or have different component arrangements.

In some embodiments, a computing device may be provided, including a memory and a processor, in which computer programs are stored. The processor may implement one or more of the following operations when the computer programs are executed by at least one processor: obtaining physiological feature data of an object within a preset time period during a medical imaging process; rendering the physiological feature data to obtain physiological feature image data corresponding to the physiological feature data, wherein the physiological feature image data may be used to express the physiological feature data in a preset displaying format; and/or storing the physiological feature image data corresponding to the medical imaging process, wherein the physiological feature image data may be configured to be used for quality impact analysis of a medical image acquired in the medical imaging process.

In some embodiments, when the computer programs are executed, the process may further implement the method including one or more of the following operations: obtaining scanning protocol data corresponding to the medical imaging process; and/or determining a rendering indication parameter from the scanning protocol data.

The obtaining physiological feature data of an object within a preset time period may include: in response to that the rendering indication parameter indicates that the physiological feature data needs to be rendered in the medical imaging process, obtaining the physiological feature data of the object within the preset time period.

In some embodiments, the processor may further implement one or more of the following operations when executing a computer program: obtaining a rendering indication parameter corresponding to the medical imaging process, the rendering indication parameter being configured to indicate whether the physiological feature data needs to be rendered in the medical imaging process; and/or storing the rendering indication parameter as a protocol parameter corresponding to the medical imaging process in scanning protocol data corresponding to the medical imaging process.

In some embodiments, when the computer programs are executed, the process may further implement the method including one or more of the following operations: obtaining the medical image corresponding to the medical imaging process; and/or displaying the medical image associatively with the physiological feature image data.

In some embodiments, when the computer programs are executed, the process may further implement the method including one or more of the following operations: determining a chronological order of scanning protocol data corresponding to the medical imaging process in a checklist; displaying the physiological feature image data in the chronological order.

The processor further implements the following operations when executing a computer program: in response to that a target event is detected within the preset time period, determining a duration time period of the target event within the preset time period; displaying target physiological feature data within the duration time period in the physiological feature data differentially from remaining data of the physiological feature image data.

In some embodiments, when the computer programs are executed, the process may further implement the method including one or more of the following operations: displaying a rendering selection controller, wherein the rendering selection controller may be used for a user to choose whether to render the physiological feature data; rendering the physiological feature data to obtain physiological feature image data corresponding to the physiological feature data, including: if a confirmation instruction input by the user based on the rendering selection controller is detected, rendering the physiological feature data to obtain the corresponding physiological feature image data.

In some embodiments, when the computer programs are executed, the process may further implement the method including one or more of the following operations: storing the physiological feature image data corresponding to the medical image, an identification of the object, an acquisition time of the medical image, and scanning protocol data corresponding to the medical image at a preset storage location.

In some embodiments, a non-transitory computer-readable storage medium may be provided, on which instructions are stored. When the instructions are executed by a processor, one or more of the following operations may be implemented: obtaining physiological feature data of an object within a preset time period during a medical imaging process; rendering the physiological feature data to obtain physiological feature image data corresponding to the physiological feature data, wherein the physiological feature image data may be used to express the physiological feature data in a preset displaying format; storing the physiological feature image data corresponding to the medical imaging process, wherein the physiological feature image data may be configured to be used for quality impact analysis of a medical image acquired in the medical imaging process.

In some embodiments, when the computer programs are executed, the process may further implement the method including one or more of the following operations: obtaining scanning protocol data corresponding to the medical imaging process; and determining a rendering indication parameter from the scanning protocol data.

The obtaining physiological feature data of an object within a preset time period may include: in response to that the rendering indication parameter indicates that the physiological feature data needs to be rendered in the medical imaging process, obtaining the physiological feature data of the object within the preset time period.

In some embodiments, the processor may further implement one or more of the following operations when executing a computer program: obtaining a rendering indication parameter corresponding to the medical imaging process, the rendering indication parameter being configured to indicate whether the physiological feature data needs to be rendered in the medical imaging process; storing the rendering indication parameter as a protocol parameter corresponding to the medical imaging process in scanning protocol data corresponding to the medical imaging process.

In some embodiments, when the computer programs are executed, the process may further implement the method including one or more of the following operations: obtaining the medical image corresponding to the medical imaging process; displaying the medical image associatively with the physiological feature image data.

In some embodiments, when the computer programs are executed, the process may further implement the method including one or more of the following operations: determining a chronological order of scanning protocol data corresponding to the medical imaging process in a checklist; displaying the physiological feature image data in the chronological order.

The processor further implements the following operations when executing a computer program: in response to that a target event is detected within the preset time period, determining a duration time period of the target event within the preset time period; displaying target physiological feature data within the duration time period in the physiological feature data differentially from remaining data of the physiological feature image data.

In some embodiments, when the computer programs are executed, the process may further implement the method including one or more of the following operations: displaying a rendering selection controller, wherein the rendering selection controller may be used for a user to choose whether to render the physiological feature data; rendering the physiological feature data to obtain physiological feature image data corresponding to the physiological feature data, including: if a confirmation instruction input by the user based on the rendering selection controller is detected, rendering the physiological feature data to obtain the corresponding physiological feature image data.

In some embodiments, when the computer programs are executed, the process may further implement the method including one or more of the following operations: storing the physiological feature image data corresponding to the medical image, an identification of the object, an acquisition time of the medical image, and scanning protocol data corresponding to the medical image at a preset storage location.

It should be noted that the user information (including but not limited to user device information, user personal information, etc.) and data (including but not limited to data used for analysis, stored data, displayed data, etc.) involved in the present disclosure are information and data authorized by the user or fully authorized by all parties.

## Claims

1. A method implemented on at least one machine each of which has at least one processor and at least one storage device for image data processing, comprising:
obtaining (510) imaging data of an object acquired from a first device (110);
obtaining (520) physiological feature data of the object acquired from a second device (120), wherein the physiological feature data at least includes synchronous physiological feature data of the imaging data;
storing (530) the imaging data of the object associatively with the physiological feature data; and **characterized in** the following steps comprised in the method:
in response to that a target event is detected within an acquisition time period of the physiological feature data, determining a duration time period of the target event;
identifying the physiological feature data based on the duration time period; and
differentially displaying or removing the physiological feature data within the duration time period.

2. The method of claim 1, wherein an acquisition time period of the synchronous physiological feature data is the same as an acquisition time period of the imaging data.

3. The method of claim 1 or 2, wherein the storing (530) the imaging data of the object associatively with the physiological feature data includes:
determining a storage path of the physiological feature data, wherein the storage path is related to the imaging data; and
storing the physiological feature data at a storage location indicated by the storage path of the physiological feature data.

4. The method of claim 3, wherein the determining a storage path of the physiological feature data includes:
determining the storage path of the physiological feature data based on related data of the imaging data and/or related data of the object, wherein
the related data of the imaging data includes at least one of a storage path of the imaging data, an imaging time of the imaging data, or scanning protocol data of the imaging data, and
the related data of the object at least includes identification information of the object.

5. The method of any one of claims 1-4, further comprising:
in response to a rendering instruction of a user, generating physiological feature image data of the object based on the physiological feature data of the object.

6. The method of claim 5, further comprising:
storing the physiological feature image data associatively with the imaging data of the object.

7. The method of claim 5, further comprising:
displaying a rendering selection controller configured to facilitate the user to input the rendering instruction; and/or
obtaining the rendering instruction through the rendering selection controller.

8. The method of any one of claims 1-7, wherein the physiological feature data includes at least one of a respiratory signal, an electrocardiogram signal, a pulse signal, a blood oxygen concentration, an electroencephalogram, an electromyography, a body temperature, a blood pressure, or an acousto-optic stimulation and a corresponding response.

9. The method of any one of claims 1-8, further comprising:
performing a quality impact analysis on the imaging data of the object stored associatively with the physiological feature data based on the physiological feature data.

10. A system for image data processing, comprising:
at least one storage device including a set of instructions; and
at least one processor configured to communicate with the at least one storage device, wherein when executing the set of instructions, the at least one processor is configured to direct the system to perform operations including:
obtaining (510) imaging data of an object acquired from a first device (110);
obtaining (520) physiological feature data of the object acquired from a second device (120), wherein the physiological feature data at least includes synchronous physiological feature data of the imaging data;
storing (530) the imaging data of the object associatively with the physiological feature data; and **characterized in** the following included operations:
in response to that a target event is detected within an acquisition time period of the physiological feature data, determining a duration time period of the target event;
identifying the physiological feature data based on the duration time period; and
differentially displaying or removing the physiological feature data within the duration time period.

11. The system of claim 10, wherein the at least one processor is further configured to direct the system to perform operations including:
displaying a rendering selection controller configured to facilitate a user to input a rendering instruction; and
in response to the rendering instruction of the user, generating physiological feature image data of the object based on the physiological feature data of the object.

12. The system of claim 11, wherein the at least one processor is further configured to direct the system to perform operations including:
storing the physiological feature image data associatively with the imaging data of the object.

13. The system of claim 10, wherein an acquisition time period of the synchronous physiological feature data is the same as an acquisition time period of the imaging data.

14. A non-transitory computer readable medium, comprising a set of instructions for data clustering, wherein when executed by at least one processor, the set of instructions direct the at least one processor to effectuate a method, the method comprising:
obtaining (510) imaging data of an object acquired from a first device (110);
obtaining (520) physiological feature data of the object acquired from a second device (120), wherein the physiological feature data at least includes synchronous physiological feature data of the imaging data;
storing (530) the imaging data of the object associatively with the physiological feature data; and **characterized in** the following steps comprised in the method:
in response to that a target event is detected within an acquisition time period of the physiological feature data, determining a duration time period of the target event;
identifying the physiological feature data based on the duration time period; and
differentially displaying or removing the physiological feature data within the duration time period.

## Patentansprüche

1. Verfahren, das auf mindestens einer Maschine implementiert ist, von denen jede mindestens einen Prozessor und mindestens eine Speichervorrichtung zur Bilddatenverarbeitung aufweist, umfassend:
Erhalten (510) von Bildgebungsdaten eines Objekts, die von einer ersten Vorrichtung (110) erfasst wurden;
Erhalten (520) von physiologischen Merkmalsdaten des Objekts, die von einer zweiten Vorrichtung (120) erfasst wurden, wobei die physiologischen Merkmalsdaten mindestens synchrone physiologische Merkmalsdaten der Bildgebungsdaten einschließen;
Speichern (530) der Bildgebungsdaten des Objekts assoziativ mit den physiologischen Merkmalsdaten; und **gekennzeichnet durch** die folgenden in dem Verfahren umfassten Schritte:
als Reaktion darauf, dass innerhalb einer Erfassungszeitspanne der physiologischen Merkmalsdaten ein Zielereignis erkannt wird, Bestimmen einer Dauerzeitspanne des Zielereignisses;
Identifizieren der physiologischen Merkmalsdaten auf der Grundlage der Dauerzeitspanne; und
differenzielles Anzeigen oder Entfernen der physiologischen Merkmalsdaten innerhalb der Dauerzeitspanne.

2. Verfahren nach Anspruch 1, wobei eine Erfassungszeitspanne der synchronen physiologischen Merkmalsdaten der gleiche ist wie ein Erfassungszeitspanne der Bildgebungsdaten.

3. Verfahren nach Anspruch 1 oder 2, wobei das Speichern (530) der Bildgebungsdaten des Objekts assoziativ mit den physiologischen Merkmalsdaten Folgendes einschließt:
Bestimmen eines Speicherpfads der physiologischen Merkmalsdaten, wobei der Speicherpfad mit den Bildgebungsdaten in Zusammenhang steht; und
Speichern der physiologischen Merkmalsdaten an einem Speicherort, der durch den Speicherpfad der physiologischen Merkmalsdaten angegeben wird.

4. Verfahren nach Anspruch 3, wobei das Bestimmen eines Speicherpfads der physiologischen Merkmalsdaten Folgendes einschließt:
Bestimmen des Speicherpfads der physiologischen Merkmalsdaten auf der Grundlage zugehöriger Daten der Bildgebungsdaten und/oder zugehöriger Daten des Objekts, wobei
die zugehörigen Daten der Bildgebungsdaten mindestens eines einschließen von einem Speicherpfad der Bildgebungsdaten, einem Bildgebungszeitpunkt der Bildgebungsdaten oder Scanprotokolldaten der Bildgebungsdaten und
die zugehörigen Daten des Objekts mindestens Identifikationsinformationen des Objekts einschließen.

5. Verfahren nach einem der Ansprüche 1-4, weiter umfassend:
als Reaktion auf eine Rendering-Anweisung eines Benutzers, Erzeugen physiologischer Merkmalsbilddaten des Objekts auf der Grundlage der physiologischen Merkmalsdaten des Objekts.

6. Verfahren nach Anspruch 5, weiter umfassend:
Speichern der physiologischen Merkmalsbilddaten assoziativ mit den Bildgebungsdaten des Objekts.

7. Verfahren nach Anspruch 5, weiter umfassend:
Anzeigen einer Rendering-Auswahlsteuereinheit, die konfiguriert ist, um dem Benutzer zu erleichtern, die Rendering-Anweisung einzugeben; und/oder
Erhalten der Rendering-Anweisung mittels der Rendering-Auswahlsteuereinheit.

8. Verfahren nach einem der Ansprüche 1-7, wobei die physiologischen Merkmalsdaten mindestens eines einschließen von einem Atemsignal, einem Elektrokardiogrammsignal, einem Pulssignal, einer Blutsauerstoffkonzentration, einem Elektroenzephalogramm, einer Elektromyographie, einer Körpertemperatur, einem Blutdruck oder einer akustooptischen Stimulation und einer entsprechenden Reaktion.

9. Verfahren nach einem der Ansprüche 1-8, weiter umfassend:
Durchführen einer Qualitätsauswirkungsanalyse an den Bildgebungsdaten des Objekts, die assoziativ mit den physiologischen Merkmalsdaten gespeichert sind, auf der Grundlage der physiologischen Merkmalsdaten.

10. System zur Bilddatenverarbeitung, umfassend:
mindestens eine Speichervorrichtung, die einen Satz von Anweisungen einschließt; und
mindestens einen Prozessor, der konfiguriert ist, um mit der mindestens einen Speichervorrichtung zu kommunizieren, wobei, wenn der Satz von Anweisungen ausgeführt wird, der mindestens eine Prozessor konfiguriert ist, um das System anzuweisen, Vorgänge durchzuführen, die Folgendes einschließen:
Erhalten (510) von Bildgebungsdaten eines Objekts, die von einer ersten Vorrichtung (110) erfasst wurden;
Erhalten (520) von physiologischen Merkmalsdaten des Objekts, die von einer zweiten Vorrichtung (120) erfasst wurden, wobei die physiologischen Merkmalsdaten mindestens synchrone physiologische Merkmalsdaten der Bildgebungsdaten einschließen;
Speichern (530) der Bildgebungsdaten des Objekts assoziativ mit den physiologischen Merkmalsdaten; und **gekennzeichnet durch** die folgenden eingeschlossenen Vorgänge:
als Reaktion darauf, dass innerhalb einer Erfassungszeitspanne der physiologischen Merkmalsdaten ein Zielereignis erkannt wird, Bestimmen einer Dauerzeitspanne des Zielereignisses;
Identifizieren der physiologischen Merkmalsdaten auf der Grundlage der Dauerzeitspanne; und
differenzielles Anzeigen oder Entfernen der physiologischen Merkmalsdaten innerhalb der Dauerzeitspanne.

11. System nach Anspruch 10, wobei der mindestens eine Prozessor weiter konfiguriert ist, um das System anzuweisen, Vorgänge durchzuführen, die Folgendes einschließen:
Anzeigen einer Rendering-Auswahlsteuereinheit, die konfiguriert ist, um einem Benutzer zu erleichtern, eine Rendering-Anweisung einzugeben; und
als Reaktion auf die Rendering-Anweisung des Benutzers, Erzeugen physiologischer Merkmalsbilddaten des Objekts auf der Grundlage der physiologischen Merkmalsdaten des Objekts.

12. System nach Anspruch 11, wobei der mindestens eine Prozessor weiter konfiguriert ist, um das System anzuweisen, Vorgänge durchzuführen, die Folgendes einschließen:
Speichern der physiologischen Merkmalsbilddaten assoziativ mit den Bildgebungsdaten des Objekts.

13. System nach Anspruch 10, wobei eine Erfassungszeitspanne der synchronen physiologischen Merkmalsdaten der gleiche ist wie ein Erfassungszeitspanne der Bildgebungsdaten.

14. Nichtflüchtiges, computerlesbares Medium, umfassend einen Satz von Anweisungen zur Datenclusterbildung, wobei der Satz von Anweisungen, wenn von mindestens einem Prozessor ausgeführt, den mindestens einen Prozessor anweist, ein Verfahren umzusetzen, wobei das Verfahren Folgendes umfasst:
Erhalten (510) von Bildgebungsdaten eines Objekts, die von einer ersten Vorrichtung (110) erfasst wurden;
Erhalten (520) von physiologischen Merkmalsdaten des Objekts, die von einer zweiten Vorrichtung (120) erfasst wurden, wobei die physiologischen Merkmalsdaten mindestens synchrone physiologische Merkmalsdaten der Bildgebungsdaten einschließen;
Speichern (530) der Bildgebungsdaten des Objekts assoziativ mit den physiologischen Merkmalsdaten; und **gekennzeichnet durch** die folgenden in dem Verfahren umfassten Schritte:
als Reaktion darauf, dass innerhalb einer Erfassungszeitspanne der physiologischen Merkmalsdaten ein Zielereignis erkannt wird, Bestimmen einer Dauerzeitspanne des Zielereignisses;
Identifizieren der physiologischen Merkmalsdaten auf der Grundlage der Dauerzeitspanne; und
differenzielles Anzeigen oder Entfernen der physiologischen Merkmalsdaten innerhalb der Dauerzeitspanne.

## Revendications

1. Procédé mis en œuvre sur au moins une machine comportant au moins un processeur et au moins un dispositif de stockage pour le traitement de données d'image, comprenant :
l'obtention (510) de données d'imagerie d'un objet acquises à partir d'un premier dispositif (110) ;
l'obtention (520) de données de caractéristiques physiologiques de l'objet acquises à partir d'un deuxième dispositif (120), dans lequel les données de caractéristiques physiologiques incluent au moins des données de caractéristiques physiologiques synchrones des données d'imagerie ;
le stockage (530) des données d'imagerie de l'objet en association avec les données des caractéristiques physiologiques ; et caractérisé dans les étapes suivantes comprises dans le procédé :
en réponse au fait qu'un événement cible est détecté pendant une période d'acquisition des données de caractéristiques physiologiques, la détermination d'une période de durée de l'événement cible ;
l'identification des données relatives aux caractéristiques physiologiques en fonction de la durée de la période ; et
l'affichage ou la suppression différentielle des données relatives aux caractéristiques physiologiques au cours de la période considérée.

2. Procédé selon la revendication 1, dans lequel la période d'acquisition des données de caractéristiques physiologiques synchrones est la même que la période d'acquisition des données d'imagerie.

3. Procédé selon la revendication 1 ou 2, dans lequel le stockage (530) des données d'imagerie de l'objet en association avec les données des caractéristiques physiologiques inclut :
la détermination d'un chemin de stockage des données relatives aux caractéristiques physiologiques, dans lequel le chemin de stockage est lié aux données d'imagerie ; et
le stockage des données des caractéristiques physiologiques à un emplacement de stockage indiqué par le chemin de stockage des données des caractéristiques physiologiques.

4. Procédé selon la revendication 3, dans lequel la détermination d'un chemin de stockage des données de caractéristiques physiologiques inclut :
la détermination du chemin de stockage des données de caractéristiques physiologiques à partir de données d'imagerie associées et/ou de données relatives à l'objet, dans lequel
les données associées aux données d'imagerie incluent au moins un élément parmi les suivants : le chemin de stockage des données d'imagerie, l'heure d'acquisition des données d'imagerie ou les données du protocole de numérisation des données d'imagerie, et
les données relatives à l'objet incluent au moins des informations d'identification de l'objet.

5. Procédé selon l'une quelconque des revendications 1-4, comprenant en outre :
en réponse à une instruction de rendu d'un utilisateur, la génération de données d'image de caractéristiques physiologiques de l'objet basées sur les données de caractéristiques physiologiques de l'objet.

6. Procédé selon la revendication 5, comprenant en outre :
le stockage des données d'image des caractéristiques physiologiques en association avec les données d'imagerie de l'objet.

7. Procédé selon la revendication 5, comprenant en outre :
l'affichage d'un dispositif de commande de sélection de rendu configuré pour permettre à l'utilisateur de saisir l'instruction de rendu ; et/ou
l'obtention des instructions de rendu via le dispositif de commande de sélection de rendu.

8. Procédé selon l'une quelconque des revendications 1-7, dans lequel les données de caractéristiques physiologiques incluent au moins un signal respiratoire, un signal électrocardiographique, un signal de pouls, une concentration d'oxygène dans le sang, un électroencéphalogramme, une électromyographie, une température corporelle, une pression artérielle ou une stimulation acousto-optique et une réponse correspondante.

9. Procédé selon l'une quelconque des revendications 1-8, comprenant en outre :
la réalisation d'une analyse d'impact sur la qualité des données d'imagerie de l'objet stockées de manière associative avec les données de caractéristiques physiologiques, en se basant sur les données de caractéristiques physiologiques.

10. Système de traitement de données d'images, comprenant :
au moins un dispositif de stockage incluant un ensemble d'instructions ; et
au moins un processeur configuré de façon à communiquer avec le au moins un dispositif de stockage, dans lequel lors de l'exécution de l'ensemble d'instructions, le au moins un processeur est configuré pour amener le système à effectuer des opérations incluant :
l'obtention (510) de données d'imagerie d'un objet acquises à partir d'un premier dispositif (110) ;
l'obtention (520) de données de caractéristiques physiologiques de l'objet acquises à partir d'un deuxième dispositif (120), dans lequel les données de caractéristiques physiologiques incluent au moins des données de caractéristiques physiologiques synchrones des données d'imagerie ;
le stockage (530) des données d'imagerie de l'objet en association avec les données des caractéristiques physiologiques ; et **caractérisé par** les opérations incluses suivantes :
en réponse au fait qu'un événement cible est détecté pendant une période d'acquisition des données de caractéristiques physiologiques, la détermination d'une période de durée de l'événement cible ;
l'identification des données relatives aux caractéristiques physiologiques en fonction de la durée de la période ; et
l'affichage ou la suppression différentielle des données relatives aux caractéristiques physiologiques au cours de la période considérée.

11. Système selon la revendication 10, dans lequel l'au moins un processeur est en outre configuré pour diriger le système afin d'effectuer des opérations incluant :
l'affichage d'un dispositif de commande de sélection de rendu configuré pour permettre à l'utilisateur de saisir une instruction de rendu ; et
en réponse aux instructions de rendu de l'utilisateur, la génération de données d'image des caractéristiques physiologiques de l'objet à partir des données des caractéristiques physiologiques de l'objet.

12. Système selon la revendication 11, dans lequel l'au moins un processeur est en outre configuré pour diriger le système afin d'effectuer des opérations incluant :
le stockage des données d'image des caractéristiques physiologiques en association avec les données d'imagerie de l'objet.

13. Système selon la revendication 10, dans lequel une période d'acquisition des données de caractéristiques physiologiques synchrones est la même que la période d'acquisition des données d'imagerie.

14. Support non transitoire lisible par ordinateur, comprenant un ensemble d'instructions pour un regroupement de données, dans lequel, lorsqu'il est exécuté par au moins un processeur, l'ensemble d'instructions amène l'au moins un processeur à effectuer un procédé, le procédé comprenant :
l'obtention (510) de données d'imagerie d'un objet acquises à partir d'un premier dispositif (110) ;
l'obtention (520) de données de caractéristiques physiologiques de l'objet acquises à partir d'un deuxième dispositif (120), dans lequel les données de caractéristiques physiologiques incluent au moins des données de caractéristiques physiologiques synchrones des données d'imagerie ;
le stockage (530) des données d'imagerie de l'objet en association avec les données des caractéristiques physiologiques ; et caractérisé dans les étapes suivantes comprises dans le procédé :
en réponse au fait qu'un événement cible est détecté pendant une période d'acquisition des données de caractéristiques physiologiques, la détermination d'une période de durée de l'événement cible ;
l'identification des données relatives aux caractéristiques physiologiques en fonction de la durée de la période ; et
l'affichage ou la suppression différentielle des données relatives aux caractéristiques physiologiques au cours de la période considérée.
